# EUROPEAN PATENT APPLICATION

(11) **EP 2 312 313 A2**
(43) Date of publication of application: **20.04.2011**
(21) Application number: 10181767.4
(22) Date of filing: 09.09.2005
(51) Int. Cl.: G01N 33/543, G01N 33/68

(54) **Immobilized probes and methods of detecting conformationally altered prion proteins**

(30) Priority: 10.09.2004 US 608541 P; 07.01.2005 US 30300
(62) Divisional of application: 05796710.1
(71) Applicant: Adlyfe, Inc., Rockville, MD 20850 (US)
(72) Inventor: Orser, Cindy S., Boulder, CO 80301 (US); Pan, Tao, Gaithersburg, MD 20878 (US)
(74) Representative: Pohlman, Sandra M.

(57) **Abstract**

The present invention provides methods of making immobilized probes that are specific for prion proteins, methods of using such probes to bind, detect, and remove prion proteins from samples, and kits for practicing the invention. The invention discloses immobilized probes that are locked into a particular, pre-determined configuration and that retain their activity and specificity even when exposed to conditions that would typically alter their activity and specificity.

SEQUENCE LISTING

<110> Adlyfe Inc.

<120> IMMOBILIZED PROBES AND METHODS OF DETECTING
      CONFORMATIONALLY ALTERED PRION PROTEINS

<130> ADL12211PCTEPD1

<140> Divisional Application based on EP 05796710.1
<141> 2005-09-2005

<150> 60/608,541
<151> 2004-09-10

<150> 11/030,300
<151> 2005-01-07

<160> 31

<170> PatentIn Ver. 3.3

<210> 1
<211> 33
<212> PRT
<213> Homo sapiens

<400> 1
Val Val Ala Gly Ala Ala Ala Ala Gly Ala Met His Lys Met Asn Thr
  1               5                  10                  15

Lys Pro Lys Met Lys His Met Ala Gly Ala Ala Ala Ala Gly Ala Val
             20                  25                  30

Val

<210> 2
<211> 19
<212> PRT
<213> Homo sapiens

<400> 2
Lys Pro Lys Thr Asn Met Lys His Met Ala Gly Ala Ala Ala Ala Gly
  1               5                  10                  15

Ala Val Val

<210> 3
<211> 14
<212> PRT
<213> Homo sapiens

<400> 3
Met Lys His Met Ala Gly Ala Ala Ala Ala Gly Ala Val Val
  1               5                  10

<210> 4
<211> 40
<212> PRT
<213> Homo sapiens

<400> 4
Asp Ala Glu Phe Arg His Asp Ser Gly Tyr Glu Val His His Gln Lys
  1               5                  10                  15

Leu Val Phe Phe Ala Glu Asp Val Gly Ser Asn Lys Gly Ala Ile Ile
             20                  25                  30

Gly Leu Met Val Gly Gly Val Val
         35                  40

<210> 5
<211> 24
<212> PRT
<213> Homo sapiens

<400> 5
Glu Val His His Gln Lys Leu Val Phe Phe Ala Glu Asp Val Gly Ser
1               5                   10                  15

Asn Lys Gly Ala Ile Ile Gly Leu
            20


<210> 6
<211> 24
<212> PRT
<213> Homo sapiens

<400> 6
Glu Val Arg His Gln Lys Leu Val Phe Phe Ala Glu Asp Val Gly Ser
1               5                   10                  15

Asn Lys Gly Ala Ile Ile Gly Leu
            20


<210> 7
<211> 11
<212> PRT
<213> Homo sapiens

<400> 7
Gly Ser Asn Lys Gly Ala Ile Ile Gly Leu Met
1               5                   10


<210> 8
<211> 27
<212> PRT
<213> Artificial Sequence

<220>
<223> Description of Artificial Sequence: Synthetic
      peptide probe

<400> 8
Lys Lys Lys Lys Lys Lys Lys Lys Lys Lys Lys Lys Lys Lys Lys Lys
1               5                   10                  15

Lys Lys Lys Lys Lys Lys Lys Lys Lys Lys Lys
            20                  25


<210> 9
<211> 23
<212> PRT
<213> Artificial Sequence

<220>
<223> Description of Artificial Sequence: Synthetic
      peptide probe

<400> 9
Gln Gln Gln Gln Gln Gln Gln Gln Gln Gln Gln Gln Gln Gln Gln Gln
1               5                   10                  15

Gln Gln Gln Gln Gln Gln Gln
            20


<210> 10
<211> 19
<212> PRT
<213> Homo sapiens

<400> 10
Lys Pro Lys Thr Asn Leu Lys His Val Ala Gly Ala Ala Ala Ala Gly
1               5                   10                  15

Ala Val Val

<210> 11
<211> 38
<212> PRT
<213> Homo sapiens

<400> 11
Met Gly Ile Leu Lys Leu Gln Val Phe Leu Ile Val Leu Ser Val Ala
1               5                   10                  15

Leu Asn His Leu Lys Ala Thr Pro Ile Glu Ser His Gln Val Glu Lys
            20                  25                  30

Arg Lys Cys Asn Thr Ala
        35

<210> 12
<211> 25
<212> PRT
<213> Homo sapiens

<400> 12
Met Ala Glu Ser His Leu Leu Gln Trp Leu Leu Leu Leu Leu Pro Thr
1               5                   10                  15

Leu Cys Gly Pro Gly Thr Ala Ala Trp
            20                  25

<210> 13
<211> 253
<212> PRT
<213> Homo sapiens

<400> 13


<210> 14
<211> 254
<212> PRT
<213> Mus sp.

<400> 14
Met Ala Asn Leu Gly Tyr Trp Leu Leu Ala Leu Phe Val Thr Met Trp
  1               5                  10                  15

Thr Asp Val Gly Leu Cys Lys Lys Arg Pro Lys Pro Gly Gly Trp Asn
             20                  25                  30

Thr Gly Gly Ser Arg Tyr Pro Gly Gln Gly Ser Pro Gly Gly Asn Arg
         35                  40                  45

Tyr Pro Pro Gln Gly Gly Thr Trp Gly Gln Pro His Gly Gly Gly Trp
     50                  55                  60

Gly Gln Pro His Gly Gly Ser Trp Gly Gln Pro His Gly Gly Ser Trp
 65                  70                  75                  80

Gly Gln Pro His Gly Gly Gly Trp Gly Gln Gly Gly Gly Thr His Asn
                 85                  90                  95

Gln Trp Asn Lys Pro Ser Lys Pro Lys Thr Asn Leu Lys His Val Ala
            100                 105                 110

Gly Ala Ala Ala Ala Gly Ala Val Val Gly Gly Leu Gly Gly Tyr Met
        115                 120                 125

Leu Gly Ser Ala Met Ser Arg Pro Met Ile His Phe Gly Asn Asp Trp
    130                 135                 140

Glu Asp Arg Tyr Tyr Arg Glu Asn Met Tyr Arg Tyr Pro Asn Gln Val
145                 150                 155                 160

Tyr Tyr Arg Pro Val Asp Gln Tyr Ser Asn Gln Asn Asn Phe Val His
                165                 170                 175

Asp Cys Val Asn Ile Thr Ile Lys Gln His Thr Val Thr Thr Thr Thr
            180                 185                 190

Lys Gly Glu Asn Phe Thr Glu Thr Asp Val Lys Met Met Glu Arg Val
        195                 200                 205

Val Glu Gln Met Cys Val Thr Gln Tyr Gln Lys Glu Ser Gln Ala Tyr
    210                 215                 220

Tyr Asp Gly Arg Arg Ser Ser Ser Thr Val Leu Phe Ser Ser Pro Pro
225                 230                 235                 240

Val Ile Leu Leu Ile Ser Phe Leu Ile Phe Leu Ile Val Gly
                245                 250


<210> 15
<211> 14
<212> PRT
<213> Artificial Sequence

<220>
<223> Description of Artificial Sequence: Synthetic
      peptide

<400> 15
Gln Arg Ser Thr Val Val Ala Arg Leu Lys Ala Ala Ala Val
1               5                   10


<210> 16
<211> 818
<212> PRT
<213> Homo sapiens

<400> 16
Met Ala Pro His Arg Pro Ala Pro Ala Leu Leu Cys Ala Leu Ser Leu
1               5                   10                  15

Ala Leu Cys Ala Leu Ser Leu Pro Val Arg Ala Ala Thr Ala Ser Arg
            20                  25                  30

Gly Ala Ser Gln Ala Gly Ala Pro Gln Gly Arg Val Pro Glu Ala Arg
        35                  40                  45

Pro Asn Ser Met Val Val Glu His Pro Glu Phe Leu Lys Ala Gly Lys
    50                  55                  60

Glu Pro Gly Leu Gln Ile Trp Arg Val Glu Lys Phe Asp Leu Val Pro
65                  70                  75                  80

Val Pro Thr Asn Leu Tyr Gly Asp Phe Phe Thr Gly Asp Ala Tyr Val
                85                  90                  95

Ile Leu Lys Thr Val Gln Leu Arg Asn Gly Asn Leu Gln Tyr Asp Leu
            100                 105                 110

His Tyr Trp Leu Gly Asn Glu Cys Ser Gln Asp Glu Ser Gly Ala Ala
        115                 120                 125

Ala Ile Phe Thr Val Gln Leu Asp Asp Tyr Leu Asn Gly Arg Ala Val
    130                 135                 140

Gln His Arg Glu Val Gln Gly Phe Glu Ser Ala Thr Phe Leu Gly Tyr
145                 150                 155                 160

Phe Lys Ser Gly Leu Lys Tyr Lys Lys Gly Gly Val Ala Ser Gly Phe
                165                 170                 175

Lys His Val Val Pro Asn Glu Val Val Val Gln Arg Leu Phe Gln Val
            180                 185                 190

Lys Gly Arg Arg Val Val Arg Ala Thr Glu Val Pro Val Ser Trp Glu
        195                 200                 205

Ser Phe Asn Asn Gly Asp Cys Phe Ile Leu Asp Leu Gly Asn Asn Ile
    210                 215                 220

His Gln Trp Cys Gly Ser Asn Ser Asn Arg Tyr Glu Arg Leu Lys Ala
225                 230                 235                 240

Thr Gln Val Ser Lys Gly Ile Arg Asp Asn Glu Arg Ser Gly Arg Ala
                245                 250                 255

Arg Val His Val Ser Glu Glu Gly Thr Glu Pro Glu Ala Met Leu Gln
            260                 265                 270

Val Leu Gly Pro Lys Pro Ala Leu Pro Ala Gly Thr Glu Asp Thr Ala
        275                 280                 285

Lys Glu Asp Ala Ala Asn Arg Lys Leu Ala Lys Leu Tyr Lys Val Ser
    290                 295                 300

Asn Gly Ala Gly Thr Met Ser Val Ser Leu Val Ala Asp Glu Asn Pro
305                 310                 315                 320

Phe Ala Gln Gly Ala Leu Lys Ser Glu Asp Cys Phe Ile Leu Asp His

                325                 330                 335

Gly Lys Asp Gly Lys Ile Phe Val Trp Lys Gly Lys Gln Ala Asn Thr
            340                 345                 350

Glu Glu Arg Lys Ala Ala Leu Lys Thr Ala Ser Asp Phe Ile Thr Lys
        355                 360                 365

Met Asp Tyr Pro Lys Gln Thr Gln Val Ser Val Leu Pro Glu Gly Gly
    370                 375                 380

Glu Thr Pro Leu Phe Lys Gln Phe Phe Lys Asn Trp Arg Asp Pro Asp
385                 390                 395                 400

Gln Thr Asp Gly Leu Gly Leu Ser Tyr Leu Ser Ser His Ile Ala Asn
                405                 410                 415

Val Glu Arg Val Pro Phe Asp Ala Ala Thr Leu His Thr Ser Thr Ala
            420                 425                 430

Met Ala Ala Gln His Gly Met Asp Asp Asp Gly Thr Gly Gln Lys Gln
        435                 440                 445

Ile Trp Arg Ile Glu Gly Ser Asn Lys Val Pro Val Asp Pro Ala Thr
    450                 455                 460

Tyr Gly Gln Phe Tyr Gly Gly Asp Ser Tyr Ile Ile Leu Tyr Asn Tyr
465                 470                 475                 480

Arg His Gly Gly Arg Gln Gly Gln Ile Ile Tyr Asn Trp Gln Gly Ala
                485                 490                 495

Gln Ser Thr Gln Asp Glu Val Ala Ala Ser Ala Ile Leu Thr Ala Gln
            500                 505                 510

Leu Asp Glu Glu Leu Gly Gly Thr Pro Val Gln Ser Arg Val Val Gln
        515                 520                 525

Gly Lys Glu Pro Ala His Leu Met Ser Leu Phe Gly Gly Lys Pro Met
    530                 535                 540

Ile Ile Tyr Lys Gly Gly Thr Ser Arg Glu Gly Gly Gln Thr Ala Pro
545                 550                 555                 560

Ala Ser Thr Arg Leu Phe Gln Val Arg Ala Asn Ser Ala Gly Ala Thr
                565                 570                 575

Arg Ala Val Glu Val Leu Pro Lys Ala Gly Ala Leu Asn Ser Asn Asp
            580                 585                 590

Ala Phe Val Leu Lys Thr Pro Ser Ala Ala Tyr Leu Trp Val Gly Thr
        595                 600                 605

Gly Ala Ser Glu Ala Glu Lys Thr Gly Ala Gln Glu Leu Leu Arg Val
    610                 615                 620

Leu Arg Ala Gln Pro Val Gln Val Ala Glu Gly Ser Glu Pro Asp Gly
625                 630                 635                 640

Phe Trp Glu Ala Leu Gly Gly Lys Ala Ala Tyr Arg Thr Ser Pro Arg
                645                 650                 655

Leu Lys Asp Lys Lys Met Asp Ala His Pro Pro Arg Leu Phe Ala Cys
            660                 665                 670

Ser Asn Lys Ile Gly Arg Phe Val Ile Glu Glu Val Pro Gly Glu Leu
        675                 680                 685

Met Gln Glu Asp Leu Ala Thr Asp Asp Val Met Leu Leu Asp Thr Trp
    690                 695                 700

Asp Gln Val Phe Val Trp Val Gly Lys Asp Ser Gln Glu Glu Glu Lys
705                 710                 715                 720

Thr Glu Ala Leu Thr Ser Ala Lys Arg Tyr Ile Glu Thr Asp Pro Ala
                725                 730                 735

Asn Arg Asp Arg Arg Thr Pro Ile Thr Val Val Lys Gln Gly Phe Glu
            740                 745                 750

Pro Pro Ser Phe Val Gly Trp Phe Leu Gly Trp Asp Asp Asp Tyr Trp
        755                 760                 765

Ser Val Asp Pro Leu Asp Arg Ala Met Ala Glu Leu Ala Ala Tyr Glu
    770                 775                 780

Arg Leu Lys Ala Thr Gln Val Ser Lys Gly Ile Arg Asp Asn Glu Arg
785                 790                 795                 800

Ser Gly Arg Ala Arg Val His Val Ser Glu Glu Gly Thr Glu Pro Glu
                805                 810                 815

Ala Met

<210> 17
<211> 146
<212> PRT
<213> Homo sapiens

<400> 17
   Met Ala Gly Pro Leu Arg Ala Pro Leu Leu Leu Leu Ala Ile Leu Ala
   1               5                   10                  15
   Val Ala Leu Ala Val Ser Pro Ala Ala Gly Ser Ser Pro Gly Lys Pro
               20                  25                  30
   Pro Arg Leu Val Gly Gly Pro Met Asp Ala Ser Val Glu Glu Glu Gly
           35                  40                  45
   Val Arg Arg Ala Leu Asp Phe Ala Val Gly Glu Tyr Asn Lys Ala Ser
       50                  55                  60
   Asn Asp Met Tyr His Ser Arg Ala Leu Gln Val Val Arg Ala Arg Lys
   65                  70                  75                  80
   Gln Ile Val Ala Gly Val Asn Tyr Phe Leu Asp Val Glu Leu Gly Arg
                   85                  90                  95
   Thr Thr Cys Thr Lys Thr Gln Pro Asn Leu Asp Asn Cys Pro Phe His
               100                 105                 110
   Asp Gln Pro His Leu Lys Arg Lys Ala Phe Cys Ser Phe Gln Ile Tyr
           115                 120                 125
   Ala Val Pro Trp Gln Gly Thr Met Thr Leu Ser Lys Ser Thr Cys Gln
       130                 135                 140
   Asp Ala
   145

<210> 18
<211> 20
<212> PRT
<213> Homo sapiens

<400> 18
Glu Glu Glu Val Ser Ala Asp Met Pro Pro Pro Pro Met Asp Ala Ser
1               5                   10                  15

Val Glu Glu Glu
            20

<210> 19
<211> 315
<212> PRT
<213> Homo sapiens

<400> 19

Met Ala Thr Leu Glu Lys Leu Met Lys Ala Phe Glu Ser Leu Lys Ser
1               5                   10                  15

Phe Gln Gln Gln Gln Gln Gln Gln Gln Gln Gln Gln Gln Gln Gln Gln
            20                  25                  30

Gln Gln Gln Gln Gln Gln Gln Gln Pro Pro Pro Pro Pro Pro Pro Pro
        35                  40                  45

Pro Pro Pro Gln Leu Pro Gln Pro Pro Pro Gln Ala Gln Pro Leu Leu
    50                  55                  60

Pro Gln Pro Gln Pro Pro Pro Pro Pro Pro Pro Pro Pro Pro Gly Pro
65                  70                  75                  80

Ala Val Ala Glu Glu Pro Leu His Arg Pro Lys Lys Glu Leu Ser Ala
                85                  90                  95

Thr Lys Lys Asp Arg Val Asn His Cys Leu Thr Ile Cys Glu Asn Ile
            100                 105                 110

Val Ala Gln Ser Val Arg Asn Ser Pro Glu Phe Gln Lys Leu Leu Gly
        115                 120                 125

Ile Ala Met Glu Leu Phe Leu Leu Cys Ser Asp Asp Ala Glu Ser Asp
    130                 135                 140

Val Arg Met Val Ala Asp Glu Cys Leu Asn Lys Val Ile Lys Ala Leu
145                 150                 155                 160

Met Asp Ser Asn Leu Pro Arg Leu Gln Leu Glu Leu Tyr Lys Glu Ile
                165                 170                 175

Lys Lys Asn Gly Ala Pro Arg Ser Leu Arg Ala Ala Leu Trp Arg Phe
            180                 185                 190

Ala Glu Leu Ala His Leu Val Arg Pro Gln Lys Cys Arg Pro Tyr Leu
        195                 200                 205

Val Asn Leu Leu Pro Cys Leu Thr Arg Thr Ser Lys Arg Pro Glu Glu
    210                 215                 220

Ser Val Gln Glu Thr Leu Ala Ala Ala Val Pro Lys Ile Met Ala Ser
225                 230                 235                 240

Phe Gly Asn Phe Ala Asn Asp Asn Glu Ile Lys Val Leu Leu Lys Ala
                245                 250                 255

Phe Ile Ala Asn Leu Lys Ser Ser Ser Pro Thr Ile Arg Arg Thr Ala
            260                 265                 270

Ala Gly Ser Ala Val Ser Ile Cys Gln His Ser Arg Arg Thr Gln Tyr
        275                 280                 285

Phe Tyr Ser Trp Leu Leu Asn Val Leu Leu Gly Leu Leu Val Pro Val
    290                 295                 300

Glu Asp Glu His Ser Thr Leu Leu Ile Leu Gly
305                 310                 315

<210> 20
<211> 17
<212> PRT
<213> Homo sapiens

<400> 20
Gln Gln Gln Gln Gln Gln Gln Gln Gln Gln Gln Gln Gln Gln Gln Gln
1               5                   10                  15

Gln

<210> 21
<211> 89

<212> PRT
<213> Homo sapiens

<400> 21
Met Gly Ile Leu Lys Leu Gln Val Phe Leu Ile Val Leu Ser Val Ala
1               5                   10                  15

Leu Asn His Leu Lys Ala Thr Pro Ile Glu Ser His Gln Val Glu Lys
            20                  25                  30

Arg Lys Cys Asn Thr Ala Thr Cys Ala Thr Gln Arg Leu Ala Asn Phe
        35                  40                  45

Leu Val His Ser Ser Asn Asn Phe Gly Ala Ile Leu Ser Ser Thr Asn
    50                  55                  60

Val Gly Ser Asn Thr Tyr Gly Lys Arg Asn Ala Val Glu Val Leu Lys
65                  70                  75                  80

Arg Glu Pro Leu Asn Tyr Leu Pro Leu
                85


<210> 22
<211> 5
<212> PRT
<213> Homo sapiens

<400> 22
Leu Ala Asn Phe Val
1               5


<210> 23
<211> 14
<212> PRT
<213> Homo sapiens

<400> 23
Val Phe Asn Ala Leu Pro Pro Pro Pro Leu Ala Asn Phe Val
1               5                   10


<210> 24
<211> 6
<212> PRT
<213> Homo sapiens

<400> 24
Phe Leu Val His Ser Ser
1               5


<210> 25
<211> 16
<212> PRT
<213> Homo sapiens

<400> 25
Ser Ser His Val Leu Phe Pro Pro Pro Pro Phe Leu Val His Ser Ser
1               5                   10                  15


<210> 26
<211> 147
<212> PRT
<213> Homo sapiens

<400> 26


<210> 27
<211> 22
<212> PRT
<213> Homo sapiens

<400> 27
Glu Ser Val Phe Val Leu Gly Ala Leu Pro Pro Pro Pro Leu Ala Gly
1               5                   10                  15

Leu Val Phe Val Ser Glu
            20


<210> 28
<211> 32
<212> PRT
<213> Homo sapiens

<400> 28
Val Ala Ala Ala Lys Leu Arg Ala Val Val Thr Ser Arg Gln Pro Pro
1               5                   10                  15

Pro Pro Gln Arg Ser Thr Val Val Ala Arg Leu Lys Ala Ala Ala Val
            20                  25                  30


<210> 29
<211> 33
<212> PRT
<213> Mus sp.

<400> 29
Val Val Ala Gly Ala Ala Ala Ala Gly Ala Val His Lys Leu Asn Thr
1               5                   10                  15

Lys Pro Lys Leu Lys His Val Ala Gly Ala Ala Ala Ala Gly Ala Val
            20                  25                  30

Val


<210> 30
<211> 4
<212> PRT
<213> Artificial Sequence

<220>
<223> Description of Artificial Sequence: Synthetic
      peptide

<400> 30

Ala Ala Ala Val
1

<210> 31
<211> 14
<212> PRT
<213> Artificial Sequence

<220>
<223> Description of Artificial Sequence: Synthetic
      peptide

<400> 31
Val Ala Ala Ala Lys Leu Arg Ala Val Val Thr Ser Arg Gln
1               5                   10

## Description

### CROSS-REFERENCE TO RELATED APPLICATIONS

This application relies on and claims the benefit of the filing date of U.S. provisional patent application 60/608,541, filed 10 September 2004, and U.S. Patent Application 11/030,300, filed 7 January 2005, the entire disclosures of which are hereby incorporated herein by reference.

### BACKGROUND OF THE INVENTION

### Field of the Invention

The present invention relates to the field of detection of misfolded proteins, such as those associated with disease states. More particularly, the present invention relates to methods, probes, and kits for detecting conformationally altered proteins and prions in a sample. In one embodiment, the conformationally altered proteins and prions are associated with amyloidogenic diseases.

### Discussion of Related Art

The conversion of normally soluble proteins into conformationally altered insoluble proteins is thought to be a causative process in a variety of diseases. In these diseases, structural conformational changes are typically required for the conversion of a normally soluble and functional protein into a defined, insoluble state. Examples of such insoluble proteins include: Aβ-peptide in amyloid plaques of Alzheimer's disease (AD) and cerebral amyloid angiopathy (CAA); α-synuclein deposits in Lewy bodies of Parkinson's disease, tau in neurofibrillary tangles in frontal temporal dementia and Pick's disease; superoxide dismutase in amylotrophic lateral sclerosis; huntingtin in Huntington's disease; and prions in Creutzfeldt-Jakob disease (CJD). For reviews, see Glenner et al., J. Neurol. Sci. 94:1-28, 1989; Haan et al., Clin. Neurol. Neurosurg. 92(4):305-310, 1990.

Often, these highly insoluble proteins form aggregates composed of non-branching fibrils with the common characteristic of a β-pleated sheet conformation. In the CNS, amyloid can be present in cerebral and meningeal blood vessels (cerebrovascular deposits) and in brain parenchyma (plaques). Neuropathological studies in human and animal models indicate that cells proximal to amyloid deposits are disturbed in their normal functions (Mandybur, Acta Neuropathol. 78:329-331, 1989; Kawai et al., Brain Res. 623:142-146, 1993; Martin et al., Am. J. Pathol. 145:1348-1381, 1994; Kalaria et al., Neuroreport 6:477-80, 1995; Masliah et al., J Neurosci. 16:5795-5811, 1996). Other studies additionally indicate that amyloid fibrils may actually initiate neurodegeneration (Lendon et al., J. Am. Med. Assoc. 277:825-831, 1997; Yankner, Nat. Med. 2:850-852, 1996; Selkoe, J. Biol. Chem. 271:18295-18298, 1996; Hardy, Trends Neurosci. 20:154-159, 1997).

In both AD and CAA, the main amyloid component is the amyloid beta protein (Aβ). The Aβ peptide, which is generated from the amyloid beta precursor protein (APP) by the action of two putative secretases, is present at low levels in the normal CNS and blood. Two major variants, Aβ₁₋₄₀ and Aβ₁₋₄₂, are produced by alternative carboxy-terminal truncation of APP (Selkoe et al., PNAS USA 85:7341-7345, 1988; Selkoe, Trends Neurosci. 16:403-409, 1993). Aβ₁₋₄₂ is the more fibrillogenic and more abundant of the two peptides in amyloid deposits of both AD and CAA. In addition to the amyloid deposits in AD cases described above, most AD cases are also associated with amyloid deposition in the vascular walls (Hardy, 1997, supra; Haan *et al.,* 1990, supra; Terry *et al.,* supra; Vinters H. V., Cerebral amyloid angiopathy, Stroke Mar-Apr;18(2):311-324, 1987; Itoh Y., et al. Subpial beta/A4 peptide deposits are closely associated with amyloid angiopathy in the elderly, Neurosci. Lett. 155(2):144-147, June 11, 1993; Yamada M., et al., Subarachnoid haemorrhage in the elderly: a necropsy study of the association with cerebral amyloid angiopathy, J. Neurol. Neurosurg. Psychiatry 56(5):543-547, May, 1993; Greenberg S.M., et al., The clinical spectrum of cerebral amyloid angiopathy: presentations without lobar hemorrhage, Neurology 43(10):2073-2079, Oct. 1993). These vascular lesions are the halhnark of CAA, which can exist in the absence of AD.

Human transthyretin (TTR) is a normal plasma protein composed of four identical, predominantly β-sheet structured units, and it serves as a transporter of the hormone thyroxin. Abnormal self assembly of TTR into amyloid fibrils causes two forms of human disease, namely senile systemic amyloidosis (SSA) and familial amyloid polyneuropathy (FAP) (Kelly, Curr. Opin. Struct. Biol. 6(1):11-17, 1996). The cause of amyloid formation in FAP is point mutations in the TTR gene; the cause of SSA is unknown. The clinical diagnosis is established histologically by detecting deposits of amyloid *in situ* in biopsy material.

To date, little is known about the mechanism of TTR conversion into amyloid *in vivo.* However, several laboratories have demonstrated that amyloid conversion can be simulated *in vitro* by partial denaturation of normal human TTR (McCutchen et al., Biochemistry 32(45):12119-12127, 1993; McCutchen and Kelly, Biochem. Biophys. Res. Comm. 197(2):415-421, 1993). The mechanism of conformational transition involves a monomeric conformational intermediate that polymerizes into liner β-sheet structured amyloid fibrils (Lai et al., Biochemistry 35(20):6470-6482, 1996). The process can be mitigated by binding with stabilizing molecules, such as thyroxin or triiodophenol (Miroy et al., Proc. Natl. Acad. Sci. USA 93(26):15051-15056, 1996.)

The precise mechanism by which neuritic plaques are formed and the relationship of plaque formation to the disease-associated neurodegenerative processes are not well-defined. The amyloid fibrils in the brains of Alzheimer's and prion disease patients are known to result in the inflammatory activation of certain cells. For example, primary microglial cultures and the THP-1 monocytic cell line are stimulated by fibrillar β-amyloid and prion peptides to activate identical tyrosine kinase-dependent inflammatory signal transduction cascades. The signaling response elicited by β-amyloid and prion fibrils leads to the production of neurotoxic products, which are in part responsible for the neurodegeneration (Combs et al., J. Neurosci. 19:928-939, 1999).

Prions are infections pathogens that cause central nervous system spongiform encephalopathies in humans and animals. Prions are distinct from bacteria, viruses, and viroids. A potential prion precursor is a protein referred to as PrP 27-30, a 28 kilodalton hydrophobic glycoprotein that polymerizes (aggregates) into rod-like filaments found as plaques in infected brains. The normal protein homologue differs from prions in that it is readily degradable, whereas prions are highly resistant to proteases. It has been suggested that prions might contain extremely small amounts of highly infectious nucleic acid, undetectable by conventional assay methods (Benjamin Lewin, "Genes IV", Oxford Univ. Press, New York, 1990, at page 1080.) The predominant hypothesis at present is that no nucleic acid component is necessary for the infectivity of prion protein.

Complete prion protein-encoding genes have since been cloned, sequence, and expressed in transgenic animals. The normal cellular prion protein, PrP^{C}, is encoded by a single-copy host gene and is normally found at the outer surface of neurons. During a post-translational process, a protein referred to as PrP^{Sc} is formed from the normal, cellular PrP isoform (PrP^{C}), and prion disease results. PrP^{Sc} is necessary for both the transmission and pathogenesis of the transmissible neurodegenerative diseases of animals and humans.

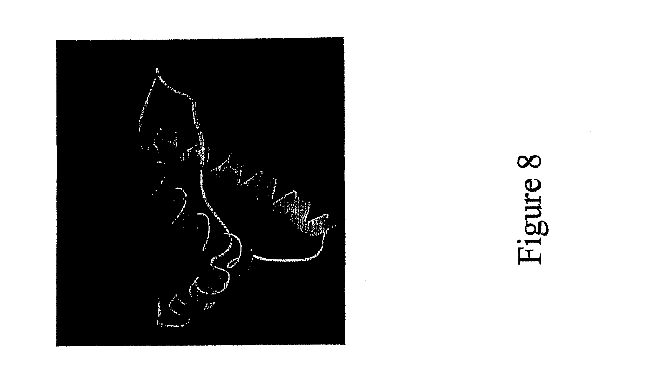

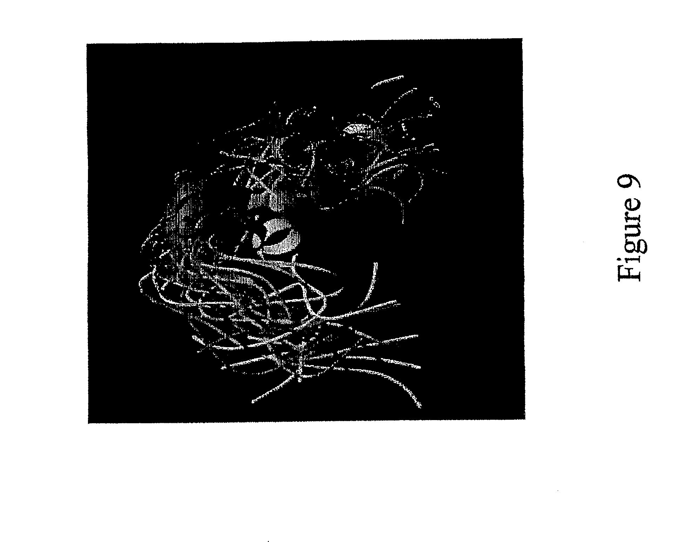

The normal prion protein (PrP^{C}) is a cell-surface metallo-glycoprotein that has mostly an α-helix and coiled-loop structure, as shown in Figure 8 herein, and is usually expressed in the central nervous and lymph systems. It is believed to serve as an antioxidant and is thought to be associated with cellular homeostasis. The abnormal form of PrP, *i.e.,* PrP^{Sc}, however, is a conformer that is resistant to proteases and has a secondary structure that contains predominantly β-sheets, as shown in Figure 9 herein. It is believed that this conformational change in secondary structure leads to aggregation and eventual neurotoxic plaque deposition in the prion disease process.

Prion-associated diseases include scrapie of sheep and goats, chronic wasting disease of deer and elk, and bovine spongiform encephalopathy (BSE) of cattle (Wilesmith and Wells, Microbiol. Immunol. 172:21-38, 1991), Four prion diseases of humans have been identified: (1) kuru, (2) Creutzfeldt-Jakob disease (CJD), (3) Gerstmann-Strassler-Scheinker Disease (GSS), and (4) fatal familial insomnia (FFI) (Gajdusek, D.C., Science 197:943-969, 1977; Medori et al. N. Engl. J. Med. 326:444-449, 1992).

Prion diseases are transmissible and insidious. For example, the long incubation times associated with prion diseases will not reveal the full extend of iatrogenic CJD for decades in thousands of people treated with cadaver-sourced human growth hormone (HGH) worldwide. The importance of detecting prions in biological products has been heightened by the possibility that bovine prions have been transmitted to humans who developed new variant Creutzfeldt-Jakob disease (nvCJD) (Chazot et al., Lancet 347:1181, 1996; Will et al., Lancet 347:921-925, 1996).

Diseases caused by prions are hard to diagnose. The disease can be latent or subclinical (abnormal prions are detectable, but symptoms are not). Moreover, normal homologues of a prion-associated protein exist in the brains of uninfected organisms, further complicating detection (Ivan Roitt et al., "Immunology", Mosby-Year Book Europe Limited, 1993, at page 15.1).

Current techniques used to detect the presence of prion-related infections rely on gross morphological changes in the brain, and on immunochemical techniques that are generally applied only after symptoms are manifest. Many of the current detection methods are antibody-based assays, or rely on affinity chromatography. They use brain tissue from dead animals, or, in some cases, capillary immunoelectrophoresis of blood samples.

Brain tissue based assays can lead to late detection and required slaughtering the animal to be tested. Prionic-Check also entails slaughtering an animal to obtain a liquefied brain tissue sample, which is subjected to an antibody using Western Blot. Although results are obtained in six to seven hours, the test does not account for the six-month lag time between PrP^{Sc} accumulation in the brain and the onset of clinical symptoms. Tonsillar biopsy sampling, and blood and cerebrospinal sampling, while accurate, can require surgical intervention and take weeks to obtain results. Electrospray ionization mass spectroscopy (ESI-MS), nuclear magnetic resonance (NMR), circular dichroism (CD), and other non-amplified structural techniques require large amounts of sample and expensive equipment that is typically located a substantial distance form the sample source.

Detection methods for conformationally altered proteins associated with the aforementioned disorders, such as AD and CAA, are also inadequate in that, like the previously mentioned prion detection techniques, they often require post-mortem tissue sampling.

Accordingly, the need exists for reliable and affordable detection methods for conformationally altered proteins and prions. Such methods should be applicable during the life of the subject at issue in order to obtain rapid diagnoses and facilitate prophylactic or remedial treatments.

### SUMMARY OF THE INVENTION

The present invention provides reliable, affordable, and safe methods for the detection of conformationally altered proteins and prions associated with a variety of diseases. Methods of the invention can be applied to obtain rapid diagnoses and facilitate prophylactic or remedial treatments. Significantly, the methods of the invention use small amounts of sample and are therefore less invasive and more readily applied than currently known diagnostic techniques. Further, methods of the invention can be used to analyze samples from a living subject and are not limited to samples obtained post mortem. In addition, they can be utilized in a manner that ensures that infectious material is not propagated during testing.

The invention overcomes many of the problems associated with prior art diagnostic techniques by using catalytic propagation to exploit conformational changes in confonnationally-altered proteins or prions associated with a particular disease process, such as transmissible spongiform encephalopathy (TSE). Catalytic propagation may be used to amplify the number of existing conformationally altered protein fragments or prions in a sample, and causes detectable aggregates to form as follows:

Upon interaction of a sample containing conformationally-altered proteins or prions with a conformational probe as defined hereinafter, the probe undergoes a conformational change and adopts the conformation of, and aggregates with, the conformationally altered proteins (which may be soluble or insoluble) or prions. The resulting aggregates, which exhibit β-sheet formation, may be readily detected using standard analytical techniques. As a result, the invention facilitates rapid and cost-effective analysis of small sample sizes and is widely applicable to tissues and body fluids from a variety of sources including, but not limited to, the brain and blood.

The invention enables detection of small amounts of disease-associated conformationally-altered proteins, such as low-density lipoprotein receptor, cystic fibrosis transmembrane regulator, Huntingtin, Aβ peptide, prions, insulin-related amyloid, hemoglobin, α-synuclein, rhodopsin, crystallins, and p53. In a preferred embodiment, methods of the invention use palindromic probes as otherwise described herein, preferably, for example, a palindromic 33-mer probe containing amino acid sequences 126-104 and 109-126 of the PrP^{Sc} protein, to detect prions in a sample. In a preferred embodiment, the probes are bound at each end to moieties that are optically distinct and detectable upon conformational conversion of the probes to a β-sheet structure.

In one embodiment, the invention provides a method for detecting conformationally altered proteins or prions in a sample comprising: (a) reacting the sample with one or more α-helix or random coil conformational probes that interact with the β-sheet conformation insoluble proteins or prions in the sample and thereby (i) undergo a conformational conversion to a predominantly β-sheet conformation, and (ii) form detectable aggregates with the β-sheet conformation insoluble proteins or prions in the sample; and (b) detecting levels of detectable aggregates, wherein levels of detectable aggregates correlate to the levels of β-sheet conformation insoluble proteins or prions in the sample and thus the infectiousness of the sample. In embodiments, the probes are immobilized on a solid support, such as a polystyrene plate, a membrane, sepharose, or polystyrene beads.

The invention also provides kits that use these methods, as well as methods of diagnosing whether a subject suffers from, or is predisposed to, a disease associated with conformationally altered proteins or prions. A kit of the present invention can comprise one or more α-helix or random coil conformational probes that interact with β-sheet conformation insoluble proteins or prions in the sample and thereby (i) undergo a conformational conversion predominantly to β-sheet conformation, and (ii) form detectable aggregates with the β-sheet conformation insoluble proteins or prions in the sample. The kit can also comprise an immobilized probe that has a particular conformation, such as a β-sheet conformation, that can interact specifically with prion disease proteins. The kit may further include moieties that bind to, or are bound to, probe termini and that are optically detectable upon conformational conversion of the probe to a predominantly β-sheet conformation, as well as instructions for using the kit, and solutions for suspending or fixing samples.

A method of diagnosing whether a subject suffers from, or is predisposed to, a disease associated with conformationally altered proteins or prions comprises: (a) obtaining a sample from the subject; (b) reacting the sample with one or more α-helix or random coil conformational probes that interact with the β-sheet conformation of insoluble proteins or prions in the sample and thereby (i) undergo a conformational conversion, preferably to a predominantly β-sheet conformation, and (ii) form detectable aggregates with the β-sheet conformation insoluble proteins or prions in the sample; and (c) detecting levels of detectable aggregates, wherein levels of detectable aggregates correlate to the amount of the β-sheet conformation insoluble proteins or prions in, and level of infectiousness of, the sample, and indicate whether the subject suffers from, or is predispose to, a disease associated with β-sheet conformation insoluble proteins or prions.

The invention also provides a method of reducing or eliminating PrP^{Sc} from a sample. The method comprises providing an immobilized probe that comprises predominantly a β-sheet conformation, contacting the immobilized probe with a sample containing or suspected of containing Prp^{Sc} under conditions and for a sufficient amount of time for the immobilized probe to bind to at least some of the PrP^{Sc} in the sample, and isolating the probe/PrP^{Sc} complexes from the rest of the sample. In embodiments, the sample is a biological sample, such as blood or plasma.

These and other aspects of the invention are described further in the following detailed description.

### BRIEF DESCRIPTION OF THE DRAWINGS

The accompanying drawings, which constitute a part of this specification, illustrate embodiments of the invention and, together with the written description, serve to explain certain principles of the invention.

Figure 1 illustrates the α-helical monomer and β-sheet dimer of a TSE conformer, along with various embodiments of the probe of the invention. The normal wild-type (wt) form of prion protein (PrP^{C}) prefers a monomeric state, while the abnormal, disease-causing form (PrP^{Sc}) prefers the multimeric (dimeric or greater) state.

Figure 2 illustrates a diagnostic analysis of a sample containing TSE protein comprised of β-sheets. The top reaction indicates the process in the presence of a mis-folded protein in a sample, while the bottom reaction indicates the process in the absence of a mis-folded protein in a sample.

Figure 3 illustrates a circular dichroism (CD) graph of a diagnostic analysis that was performed in accordance with the invention and that used poly-L-lysine (20 micromolar (µM), 52,000 molecular weight (MW)) as a peptide model.

Figure 4 illustrates an absorbance graph of a diagnostic analysis that was performed using poly-L-lysine (70 µM, 52, 000 MW) as a peptide model.

Figure 5 illustrates the results from Figure 3, that used poly-L-lysine (70 µM, 52,000 MW) as a peptide model and the effect of pH and temperature on conformational change.

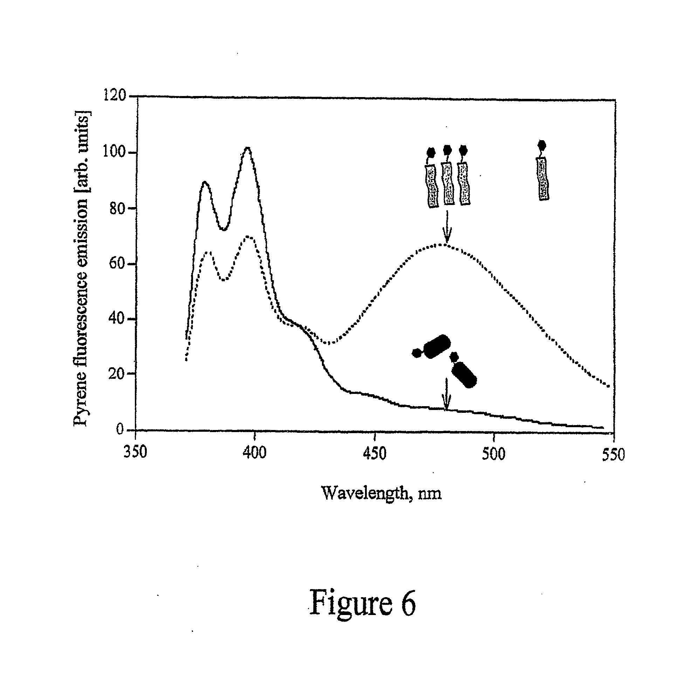

Figure 6 illustrates a spectroscopic analysis that used pyrene as a fluorescent probe in proximal and distal locations in an alpha helical bundle structure that underwent conformational change.

Figure 7 illustrates energy changes associated with conformational changes in proteinaceous material or prions.

Figure 8 illustrates the alpha-helix and loop structure of PrP^{C}.

Figure 9 illustrates the predominantly β-sheet secondary structure of PrP^{Sc}.

Figure 10 illustrates a palindromic 33-mer probe that can be used in the methods of the present invention, represented by its sequence and in a graphical form attached to pyrene labels at each end.

Figure 10A depicts the linear sequence of a 33-mer probe (SEQ ID NO:1) and the 19-mer (SEQ ID NO:2) and 14-mer (SEQ ID NO:3) that it comprises, with palindromic sequences underlined. In this figure, a probe specific for human prion protein or hamster prion protein is depicted. A probe for the murine sequence is identical except that human methionine residues are substituted with valine and leucine residues in the murine sequences (see SEQ ID NOs: 29, 10, and 3).

Figure 10B depicts the folded sequence, demonstrating the parallel palindrome that can form from the sequence and how pyrene molecules present on both ends of the peptide can form an excimer structure,

Figure 11 illustrates a circular dichroism graph of three distinct common conformational forms that proteins and peptides can assume (source: Woody, R.W., In Circular Dichroism and the Conformational Analysis of Biomolecules, Fasman, G.D., Ed., Plenum Press, New York, 1996, pages 25-69.

Figure 12 illustrates a circular dichroism graph of a diagnostic analysis that was performed in aqueous conditions in accordance with the invention and that used a palindromic 33-mer probe and the 14-mer and the 19-mer amino acid sequences that make it up (these three sequences are set forth in Figure 10).

Figure 13 illustrates a variation of the spectroscopic analysis of Figure 6, in which spectroflourometric data of a diagnostic analysis that was performed using a palindromic 33-mer probe (SEQ ID NO:1, See Figure 10) that had pyrene attached to both ends. The spectral scans in the monomer (open) conformation yielded a strikingly fluorescent spectrum that had a maximum emission between 370 nm and 385 nm, while the excited dimer or excimer state of the pyrene-labeled peptide had an emission maximum between 475 nm and 510 nm.

Figure 14 illustrates a spectroscopic analysis in which pyrene was used as a fluorophor, the excitation wavelength was around 350 nm, and the observation wavelength was around 365 nm to 600 nm. The normal emission of monomer pyrene following excitation (simple fluorescence) was recorded as the maximum wavelength detected between about 370 nm and 385 nm.

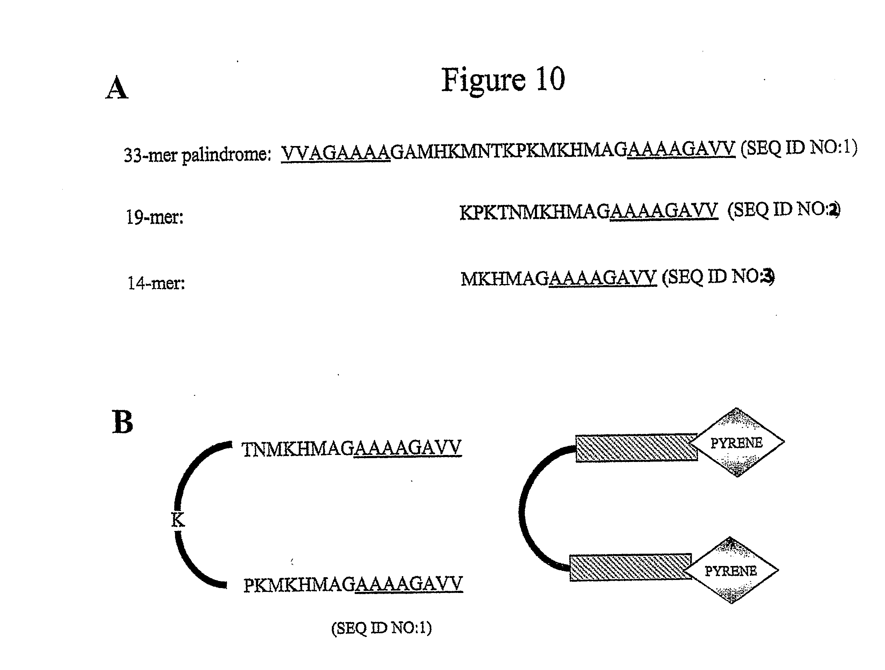

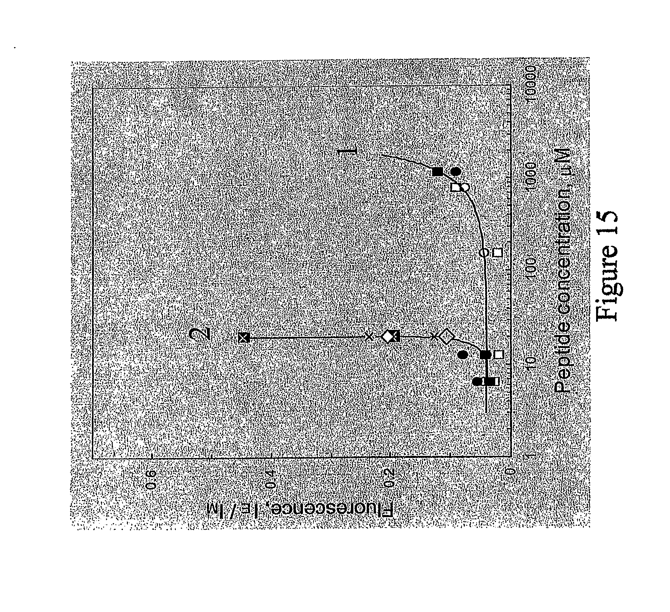

Figure 15 illustrates the ratio of excimer formation (I_{E}) to monomer formation (I_{M}) in a diagnostic analysis that used the palindromic 33-mer probe depicted in Figure 10, at different concentrations of probe. One expects to see minimum solubility of a protein when the conditions are near its isoelectric point, and that is what was observed where conditions (2) approach the isoelectric point of the 33-mer peptide: it aggregates with itself because it has dramatically reduced solubility under these conditions as compared to (1). In this example, electrostatic interactions (pI= 10) trigger self association under extremely low concentrations (10 µM) of peptide at the isoelectric point of the peptide. The following legend applies to Figure 15:

### 1. pH 6-8, salt concentration ranging from 100-500 mM KCI

### 2. pH 10-11, salt concentration ranging from 100-500 mM KCI

Figure 16 illustrates an associative curve for conformation changes in a diagnostic analysis that used a palindromic 33-mer probe (SEQ ID NO:1), a 19-mer (SEQ ID NO:2), and a 14-mer (SEQ ID NO:3) (see Figure 10) under various conditions to determine the optimal parameters associated with the transformation from random coil to β-sheet.

Figure 17 shows results from the experiment described in Example 6, where the fluorescence of a complex of prion protein and the 33-mer probe was measured as a function of time. The complex substantially dissociated over time (1 hour - 24 hours).

Figure 18 (A)-(C) illustrate fluorescence spectra of target peptide (520 mM) in the presence of infected brain homogenate (1), healthy brain homogenate (2), and peptide alone (3) in TRIS:TFE (1:1) solvent. The data were obtained for 0.01% brain homogenate from hamster ("A"; 270 pg/ml), sheep ("B"; 60 pg/ml), and elk ("C"; 6 pg/ml).

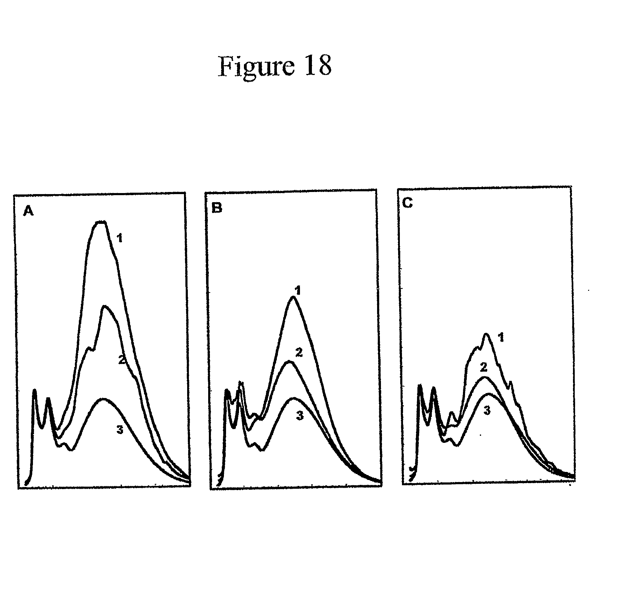

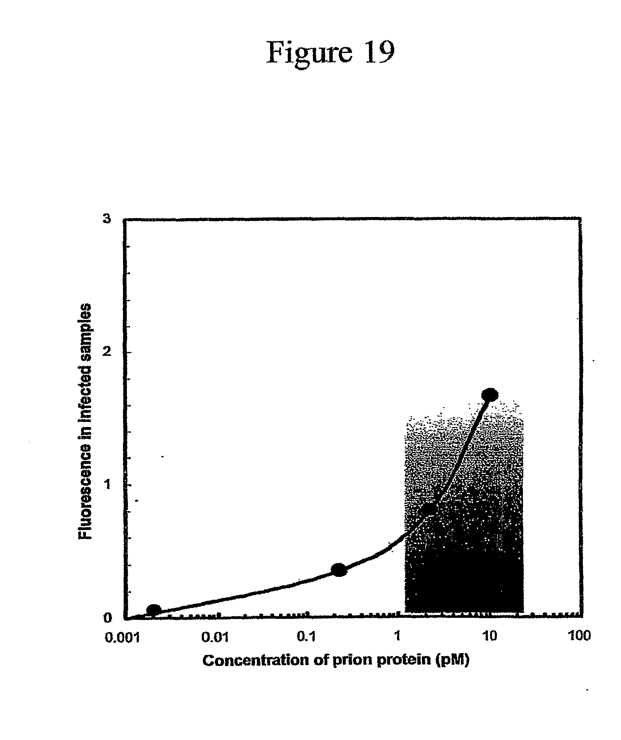

Figure 19 illustrates a calibration curve of a fluorescent diagnostic analysis conducted in accordance with the invention. The line connecting the solid circles represents the detection curve obtained using the probes and systems of the present invention. The area in the grey region (from about 1 to about 11 pM) and to the right of the grey region represents the sensitivity of presently available commercial detection kits. The data illustrated in this Figure evidence that the present invention is more than two orders of magnitude more sensitive than the validated tests in use today, without any optimization. *Prion Infectivity*: 1 IU - 3 fM:= 200,000 PrP. The prion protein concentration was determined using the capillary immunoelectrophoresis method of Dr. Schmerr (Schmerr et al., J. Chromatogr. A., 853(1-2):207-214, August 20, 1999). The data are taken from Figure 18.

Figure 20 depicts fluorescence spectra of two probes according to the invention, which have been independently immobilized to a polystyrene plate. The two probes were independently immobilized at a different pH: the probe labeled "α-helix" was immobilized at pH 7.4, whereas the probe labeled "β-sheet" was immobilized at pH 5.0. The figure shows spectra obtained at pH 7.0, and indicates that immobilization of a peptide at a given conformation locks the peptide in that conformation.

Figure 21 depicts graphs showing that immobilized target peptides retain the ability to bind PrP^{Sc} or PrP^{C}.

Figure 21A depicts absorbance of complexes of immobilized β-sheet conformation probes of the invention with either PrP^{C} or PrP^{Sc}, and indicates that the immobilized probes preferentially bind to PrPSc in a dose-dependent manner.

Figure 21B depicts a bar graph showing that immobilized target peptides distinguish between PrP^{Sc} and PrP^{C},

Figure 22 depicts a graph of results of competition assays using bound mis-folded prion protein probe and either normal prion protein or mis-folded prion protein.

### DETAILED DESCRIPTION OF EMBODIMENTS OF THE INVENTION

Reference will now be made in detail to various exemplary embodiments of the invention, examples of some of which are illustrated in the accompanying drawings.

As used herein, the following terms have the following respective meanings.

"Amyloidogenic diseases" are diseases in which amyloid plaques or amyloid deposits are formed in the body. Amyloid formation is found in a number of disorders, such as diabetes, AD, scrapie, GSS, BSE, CJD, chronic wasting disease (CWD), and related transmissible spongiform encephalopathies (TSEs).

TSEs are fatal neurodegenerative diseases that include such human disorders as CJD, kuru, FFI, and GSS. Animal forms of TSE include scrapie in sheep, CWD in deer and elk, and BSE in cattle. These diseases are characterized by the formation and accumulation in the brain of an abnormal proteinase K resistant isoform (PrP-res) of a normal protease-sensitive, host-encoded prion protein (PrP-sen). PrP-res is formed from PrP-sen by a post-translational process involving conformational changes that convert the PrP-sen into a PrP-res molecular aggregate having a higher β-sheet content. The formation of these macromolecular aggregates of PrP-res is closely associated with TSE-mediated brain pathology, in which amyloid deposits of PrP-res are formed in the brain, which eventually becomes "spongiform" (filled with holes).

TSE diseases appear to be transmitted by exposure to an unusual agent, for example by ritual cannibalism in the Foret people of New Guinea, or feeding of animal parts to cattle in BSE. Iatrogenic CJD has also been caused by administration of human growth hormone derived from cadaveric pituitaries, transplanted dura matter and corneal grafts, and by exposure of surgeons to affected tissue during neurological procedures.

The presence of a native prion protein (PrP) has been shown to be essential to pathogenesis of TSE. The cellular protein PrP-sen is a sialoglycoprotein encoded by a gene that, in humans, is located on chromosome 20. The PrP gene is expressed in both neural and non-neural tissues, with the highest concentration of its mRNA being found in neurons. The translation product of the PrP gene consists of 253 amino acids in humans, 254 amino acids in hamsters and mice, 264 amino acids in cows, and 256 amino acids in sheep (all of these sequences are disclosed in U.S. Patent No. 5,565,186, which describes methods of making transgenic mice that express species-specific PrP). In prion protein related encephalopathies, the cellular PrP-sen is converted into the altered PrP-res. PrP-res is distinguishable from PrP-sen in that PrP-res aggregates (Caughey and Chesebro, Trends Cell Biol. 7:56-62, 1997); is at least partially resistant to proteinase K digestion (only approximately the N-terminal 67 amino acids are removed by proteinase K digestion under conditions in which PrP-sen is completely degraded) (Prusiner et al., Sem. Virol. 7:159-173, 1996); and has, as compared to PrP-sen, less α-helical structure and more β-sheet structure (Pan et al., Proc. Natl. Acad. Sci. USA 90:10962-10966, 1993).

If PrP-sen is not expressed in the brain tissue of animal recipients of scrapie-infected neurografts, no pathology occurs outside the graft, demonstrating that PrP-res and PrP-sen are both required for the pathology (Brander et al., Nature 379:339-343, 1996). The long latency period between infection and the appearance of disease (months to decades, depending on species) has prompted the development of a cell-free *in vitro* test, in which PrP-res induces the conversion of PrP-sen to PrP-res (Kockisko et al., Nature 370:471-474, 1994; see also Prusiner *et al.,* WO 97/16728, published May 9, 1997). These *in vivo* and *in vitro* observations indicated that direct interactions between PrP-res and PrP-sen form PrP-res and promote TSE pathogenesis.

Small synthetic peptides containing certain PrP sequences have previously been shown to spontaneously aggregate to form fibrils with a high degree of β-sheet secondary structure of the type seen in the insoluble deposits in TSE afflicted brains (Gasset et al., Proc. Natl. Acad. Sci. USA 89:10940-10944, 1992; Come et al, Proc. Natl. Acad. Sci. USA 90:5959-5963, 1993; Forloni et al., Nature 362:543-546, 1993; Hope et al., Neurodegeneration 5:1-11, 1996). Moreover, other synthetic PrP peptides have been shown to interact with PrP-sen molecules to form an aggregated complex with increased protease-resistance (Kaneko et al, Proc. Natl. Acad. Sci. USA 92:11160-11164, 1995; Kaneko et al., J. Mol. Biol. 270:574-586, 1997).

"Conformationally altered proteins" include any protein that has a three dimensional conformation associated with a disease and that is different from the three dimensional conformation it assumes when not associated with a disease. The conformationally altered protein may cause the disease, may be a factor in a symptom of the disease, or may appear in a sample or *in vivo* as a result of other factors. According to the definition, a conformationally altered protein can have a single amino acid sequence that results in at least two conformations, one associated with disease and one not. Conformationally altered proteins are generally in the form of insoluble proteins exhibiting β-sheet formation, and are analyzed in the present invention.

The following is a non-limiting list of diseases associated with conformationally altered proteins, followed parenthetically by the associated conformationally altered protein: Alzheimer's Disease (APP, Aβ peptide, α1-antichymotrypsin, tau, non-Aβ component, presenilin 1, presenilin 2, apoE); prion diseases, CJD, scrapie, and BSE (PrP^{Sc}); ALS (SOD and neurofilament); Pick's disease (Pick body); Parkinson's disease (α-synuclein in Lewy bodies); frontotemporal dementia (tau in fibrils); diabetes type II (amylin); multiple myeloma - plasma cell dyscrasias (IgGL-chain); familial amyloidotic polyneuropathy (transthyretin); medullary carcinoma of thyroid (procalcitonin); chronic renal failure (β₂-microglobulin); congestive heart failure (atrial natriuretic factor); senile cardiac and systemic amyloidosis (transthyretin); chronic inflammation (serum amyloid A); atherosclerosis (ApoA1); familial amyloidosis (gelsolin); Huntington's disease (Huntingtin).

An "insoluble protein" includes any protein associated with an amyloidogenic disease, including, but not limited to, any of the proteins identified in the preceding paragraph. Insoluble proteins generally exhibit β-sheet formation in the aggregate.

"PrP protein", "PrP", and the like are used interchangeably herein and shall mean both the infections particle form PrP^{Sc}, known to cause diseases (spongiform encephalopathies) in humans and animals, and the non-infectious form PrP^{C}, which, under appropriate conditions, is converted to the infectious PrP^{Sc} form.

The terms "prion", "prion protein", "PrP^{Sc} protein", and the like are used interchangeably herein to refer to the infectious PrP^{Sc} form of a PrP protein. "Prion" is a contraction of the words "protein" and "infection". Particles are comprised largely, if not exclusively, of PrP^{Sc} molecules encoded by a PrP gene. Prions are distinct from bacteria, viruses, and viroids. Known prions infect animals and cause scrapie, a transmissible, degenerative disease of the nervous system of sheep and goats, as well BSE (or mad cow disease) and feline spongiform encephalopathy of cats. Four prion diseases known to affect humans are (1) kuru, (2) CJD, (3) GSS, and (4) FFI. As used herein, "prion" includes all forms of prions causing all or any of these diseases or others in any animals used, and in particular in humans and domesticated farm animals.

The term "PrP gene" is used herein to describe genetic material that expresses proteins that include known polymorphisms and pathogenic mutations. The term "PrP gene" refers generally to any gene of any species that encodes any form of a prion protein. The PrP gene can be from any animal, and includes all polymorphisms and mutations thereof, it being recognized that the terms include other such PrP genes that are yet to be discovered. The protein expressed by such a gene can assume either a PrP^{C} (non-disease) or PrP^{Sc} (disease) form.

A "peptidomimetic" is a biomolecule that mimics the activity of another biologically active peptide molecule.

"Protein" refers to any polymer of two or more individual amino acids (whether or not naturally occurring) linked via a peptide bond, which occurs when the carboxyl carbon atom of the carboxylic acid group bonded to the α-carbon of one amino acid (or amino acid residue) becomes covalently bound to the amino nitrogen atom of amino group bonded to the α-carbon of an adjacent amino acid. These peptide bonds, and the atoms comprising them (*i.e*., α-carbon atoms, carboxyl carbon atoms and their substituent oxygen atoms, and amino nitrogen atoms and their substituent hydrogen atoms) form the "polypeptide backbone" of the protein. In simplest terms, the polypeptide backbone shall be understood to refer to the amino nitrogen atoms, α-carbon atoms, and carboxyl carbon atoms of the protein, although two or more of these atoms (with or without their substituent atoms) may also be represented as a pseudoatom. Indeed, any representation of a polypeptide backbone that can be used in a functional site descriptor as described herein will be understood to be included within the meaning of the term "polypeptide backbone".

The term "protein" is understood to include the terms "polypeptide" and "peptide" (which, at times, may be used interchangeably herein) within its meaning. In addition, proteins comprising multiple polypeptide subunits (*e.g*., DNA polymerase III, RNA polymerase II) or other components (for example, an RNA molecule, as occurs in telomerase) will also be understood to be included within the meaning of "protein" as used herein. Similarly, fragments of proteins and polypeptides are also within the scope of the invention and may be referred to herein as "proteins".

"Conformation" or "conformational constraint" refers to the presence of a particular protein conformation, for example, an α-helix, parallel and antiparallel β-strands, leucine zipper, zinc finger, etc. In addition, conformational constraints can include amino acid sequence information without additional structural information. As an example, "--C--X--X--C--" is a conformational constraint indicating that two cysteine residues must be separated by two other amino acid residues, the identities of each of which are irrelevant in the context of this particular constraint. A "conformational change" is a change from one conformation to another.

The exact mechanism by which the sequence of a protein encodes the proper fold is unknown. In order to achieve the native state encoded by the fold, the protein molecule must convert to a unique conformation selected from many alternatives. Functional proteins are typically soluble and can adopt a variety of structures including coils and ordered elements. Ordered elements include the alpha helix predominant in proteins such a myoglobin and hemoglobin. During the human aging process, in some proteins the soluble structure (*e.g.*, alpha helical regions) becomes conformationally altered into beta sheet structures that undergo aggregation associated with loss of function.

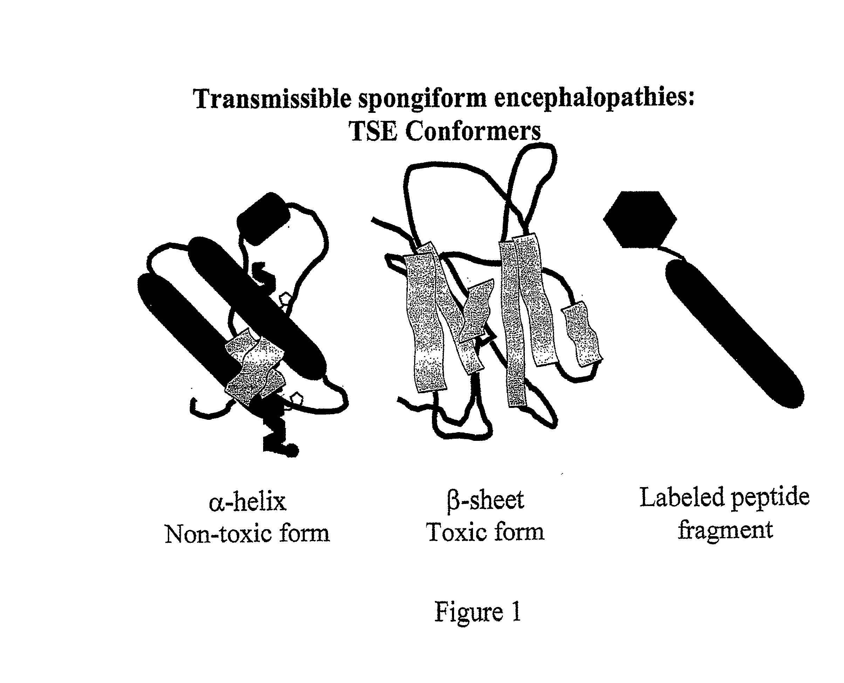

There are at least twenty proteins that are associated with human disease when they adopt a conformationally altered state, and some of these have been described previously. Figure 1 illustrates both the alpha-helical monomer and the beta-sheet dimer forms of a TSE conformer. The normal wild-type (wt) form of prion protein (PrP^{C}) prefers a monomeric state, while the abnormal, disease-causing form (PrP^{Sc}) more readily takes on a multimeric state.

Protein structures can be determined by a variety of experimental or computational methods, several of which are described below. Protein structure can be assessed experimentally by any method capable of producing at least low resolution structures. Such methods currently include X-ray crystallography and nuclear magnetic resonance (NMR) spectroscopy. X-ray crystallography is one method for protein structural evaluation, and is based on the diffraction of X-ray radiation of a characteristic wavelength by electron clouds surrounding the atomic nuclei in the crystal. X-ray crystallography uses crystals of purified biomolecules (but these frequently include solvent components, co-factors, substrates, or other ligands) to determine near atomic resolution of the atoms making up the particular biomolecule. Techniques for crystal growth are known in the art, and typically vary from biomolecule to biomolecule. Automated crystal growth techniques are also known.

Nuclear magnetic resonance (NMR) currently enables determination of the solution conformation (rather than crystal structure) of biomolecules. Typically, only small molecules, for example proteins of less than about 100-150 amino acids, are amenable to this technique. However, recent advances have lead to the experimental elucidation of the solution structures of large proteins, using such techniques as isotopic labeling. The advantage of NMR spectroscopy over X-ray crystallography is that the structure is determined in solution, rather than in a crystal lattice, where lattice neighbor interactions can alter the protein structure. The disadvantage of NMR spectroscopy is that the NMR structure is not as detailed or as accurate as a crystal structure. Generally, biomolecule structures determined by NMR spectroscopy are of moderate resolution compared to those determined by crystallography.

Other techniques useful in studying biomolecule structures include circular dichroism (CD), fluorescence, and ultraviolet-visible absorbance spectroscopy. See, for example, Physical Biochemistry: Applications to Biochemistry and Molecular Biology, 2nd ed., W. H. Freeman & Co., New York, NY, 1982, for descriptions of these techniques.

"Equivalent" refers to a protein having an amino acid sequences that is similar to the amino acid sequence of the protein to be analyzed, but has at least one, but fewer than 5 (*e.g*., 3 or fewer) differences in the amino acid sequence, such as by way of substitutions, additions, or deletions. Thus, the substitution of one or more amino acids in a given sequence that does not substantially change the basic function of the protein and the particular residue position within its use in context, is an equivalent for purposes of describing the present invention.

"Homology", "homologs of", "homologous", "identity", or "similarity" refers to sequence similarity between two polypeptides, with identity being a more strict comparison. Homology and identity can each be determined by comparing a position in each sequence that may be aligned for purposes of comparison. When a position in the compared sequence is occupied by the same amino acid, then the molecules are identical at that position. A degree of identity of amino acid sequences is a function of the number of identical amino acids at positions shared by the amino acid sequences. A degree of homology or similarity of amino acid sequences is a function of the number of amino acids, *i.e*., structurally related, at positions shared by the amino acid sequences. An "unrelated" or "non-homologous" sequence shares 40% or less identity, though preferably less than 25% identity, with one of the sequences used in the present invention. Related sequences share more than 40% identity, preferably at least about 50% identity, more preferably at least about 70% identity, even more preferably at least about 90% identity, more preferably at least about 99% identity.

The term "percent identical" refers to sequence identity between two amino acid sequences. Identity can be determined by comparing a position in each sequence that is aligned for purposes of comparison. When an equivalent position in one compared sequences is occupied by the same amino acid in the other at the same position, then the molecules are identical at that position; when the equivalent site occupied by the same or a similar amino acid residue (*e.g*., similar in stearic and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as part of the GCG sequence analysis package (University of Wisconsin, Madison, WI), and can be used with, *e.g*., default settings. ENTREZ is available through the National Center for Biotechnology Information, National Library of Medicine, NIH, Bethesda, MD). In one embodiment, the percent identity of two sequences can be determined by the GCG program with a gap weight of 1, *e.g*., each amino acid gap is weighted as if it were a single amino acid mismatch between the two sequences. Other techniques for determining sequence identity are well known and described in the art.

The term "interact" as used herein is meant to include detectable interactions (*e.g*., biochemical interactions) between molecules, such as protein-protein, protein-nucleic acid, nucleic acid-nucleic acid, protein-small molecule, or nucleic acid-small molecule interactions.

The term "homolog of an insoluble protein" includes all amino acid sequences that are encoded by a homolog of an insoluble protein gene, and all amino acid sequences that are equivalent or homologous to such sequence. Therefore, "homolog of an insoluble protein" includes proteins that are scored as hits in the Pfam family. To the identify the presence of an "insoluble protein" domain in a protein sequence, and make the determination that a polypeptide or protein of interest has a particular profile, the amino acid sequence of the protein can be searched against one of several databases (SwissProt, PIR, for example) using various default parameters (http://www.sangetr.ac.uk?Software/Pfam?HMM_search). For example, the hmmsf program, which is available as part of the HM_MER package of search programs, is a family-specific default program for MILPAT0063 and a score of 15 is the default threshold score for determining a hit. Alternatively, the threshold score for determining a hit can be lowered (*e.g*., to 8 bits). A description of the Pfam database can be found in Sonham et al., Proteins 28(3):405-420, 1997, and a detailed description of HMMs can be found, for example, in Gribskov et al., Meth. Enzymol. 183:146-159, 1990; Gribskov et al., Proc. Natl. Acad. Sci. USA 84:4355-4358, 1987; Krogh et al., J. Mol. Biol, 234:1501-1531, 1994; and Stultz et al., Protein Sci. 2:305-314, 1993, the contents of which are incorporated herein by reference.

"Test specimen" is a sample of material to be tested and is equivalent in meaning to, and thus used interchangeably with "sample". The sample may be prepared from tissue (*e.g*. a portion of ground meat, an amount of tissue obtained by a biopsy procedure, blood or a fraction of blood, such as plasma) by homogenization in a glass homogenizer or may be used directly as obtained. The amount of sample can be any amount suitable for the application for which the invention is used. For example, if blood or a blood fraction is used, it can be about 1 µl, about 100 µl, about 1 ml, about 10 ml., about 100 ml., about one liter (or one pint), or more. In some applications of the invention, large volumes of blood or blood products can be used as a sample, including amounts greater than one liter (or one pint). When solid tissue is the source of the sample, the sample should be between about 1 mg and 1 gm, preferably between 10 mg and 250 mg, ideally between 20 and 100 mg. The material to be sampled may be suspended in a suitable solvent, preferably phosphate-buffered saline at a pH between 7.0 and 7.8. The solvent may contain a detergent, such as Triton X-100, SDS, or sarkosyl. Homogenization is performed for a number of excursions of the homogenizer, preferably between 10 and 25 strokes; ideally between 15 and 20 strokes. The suspended sample is preferably centrifuged at between 100 and 1,000 g for 5-10 minutes and the supernatant material sampled for analysis. In some samples, it might be preferable to treat the supernatant material with an additional reagent, such as phosphotungstic acid according to the procedure described by Safar et al., Nature Medicine 4:1157-1165, 1998, and as modified by Wadsworth et al., Lancet 358:171-180, 2001.

The amount of sample to be tested is based on a determination of the protein content of the supernatant solution as measured by the procedure described by Bradford. Preferably, this corresponds to between 0.5 and 2 mg of protein.

In addition to the procedure described above for tissue material, test samples may be obtained from serum, pharmaceutical formulations that might contain products of animal origin, spinal fluid, saliva, urine, or other bodily fluids. Liquid samples may be tested directly or may be subjected to treatment with agents such as phosphotungstic acid, as described above.

"Conformational probes" are preferably peptides that have amino acid sequences that are similar to, and more preferably identical to, some of those in the target protein and that also have the potential to undergo conformational alterations to produce β-sheet formation when complexed with the target protein (insoluble protein). Such alteration typically leads to a β-sheet structure not normally evidenced by the probe. Ideally, a probe has a palindromic structure with two amino acid sequences derived from the target protein. Preferred α-helix or random coil conformational probes (*i.e*., probes that exhibit α-helix or random coil conformation in solution) useful in the instant invention include the following:

a palindromic 33-mer comprising amino acid sequences that are identical to amino acids 122-104 and 109-122 of the PrP^{Sc} protein (SEQ ID NO:13 and 14)
(SwissProt PO4156; Pfam ID Prion Pf00377 & 03991)
VVAGAAAAGAVHKLNTKPKLKHVAGAAAAGAVV (murine) (SEQ ID NO:29)
VVAGAAAAGAMHKMNTKPKMKHMAGAAAAGAVV (human) (SEQ ID NO:1);

a palindromic 33-mer comprising amino acid sequences that are equivalent to amino acids 122-104 and 109-122 of the PrP^{Sc} protein (SEQ ID NO:13 and 14)
(SwissProt PO4156; Pfam ID Prion Pf00377 & 03991)
VVAGAAAAGAVHKLNTKPKLKHVAGAAAAGAVV (murine) (SEQ ID NO:29)
VVAGAAAAGAMHKMNTKPKMKHMAGAAAAGAVV (human) (SEQ ID NO:1);

a palindromic 33-mer comprising amino acid sequences that are between about 70% to about 90% identical to amino acids 122-104 and 109-122 of the PrP^{Sc} protein (SEQ ID NO:13 and 14) (SwissProt PO4156; Pfam ID Prion Pf00377 & 03991) VVAGAAAAGAVHKLNTKPKLKHVAGAAAAGAVV (murine) (SEQ ID NO:29)
VVAGAAAAGAMHKMNTKPKMKHMAGAAAAGAVV (human) (SEQ ID NO:1);

a probe comprising amino acid sequences that are identical to amino acids 1-40 of the Aβ peptide (Nref 00111747; human)
DAEFRHDSGYEVHHQKLVFFAEDVGSNKGAIIGLMVGGVV (SEQ ID NO:4);

a probe comprising amino acid sequences that are equivalent to amino acids 1-40 of the Aβ peptide (Nref 00111747; human)
DAEFRHDSGYEVHHQKLVFFAEDVGSNKGAIIGLMVGGVV (SEQ ID NO:4);

a probe comprising amino acid sequences that are between about 70% to about 90% identical to amino acids 1-40 of the Aβ peptide (Nref 00111747; human)
DAEFRHDSGYEVHHQKLVFFAEDVGSNKGAIIGLMVGGVV (SEQ ID NO:4);

a probe comprising amino acid sequences that are identical to amino acids 11-34 of the Aβ peptide (Nref 00111747; human)
EVHHQKLVFFAEDVGSNKGAIIGL (SEQ ID NO:5);

a probe comprising amino acid sequences that are identical to amino acids 11-34 of the Aβ peptide (Nref 00111747; human), but with residue H13 substituted with R to reduce metal ion interactions and to increase the solubility of the peptide
EVRHQKLVFFAEDVGSNKGAIIGL (SEQ ID NO:6);

a probe comprising amino acid sequences that are identical to amino acids 25-35 of the Aβ peptide (Nref00111747; human)
GSNKGAIIGLM (SEQ ID NO:7);

a probe that has a helix-loop-helix conformation found in polylysine and an amino acid sequence that is at least 10 amino acid residues in length and is equivalent or homologous to KKKKKKKKKKKKKKKKKKKKKKKKKKK (27-mer) (SEQ ID NO:8);

a probe that has a conformation found in polyglutamine and an amino acid sequence that is equivalent or homologous to QQQQQQQQQQQQQQQQQQQQQQQ (23-mer) (SEQ ID NO:9);

a probe comprising amino acid sequences that are homologous to amino acids 104-122 or wild-type (wt) TSE (human Nref 00130350)
KPKTNLKHVAGAAAAGAVV (SEQ ID NO:10);

a probe comprising amino acid squences that are equivalent to amino acids 104-122 of wild-type (wt) TSE (human Nref 00130350)
KPKTNLKHVAGAAAAGAVV (SEQ ID NO:10);

a probe comprising amino acid sequences that are between about 70% to about 90% identical to amino acid sequences 104-122 of wild-type (wt) TSE (human Nref 00130350)
KPKTNLKHVAGAAAAGAVV (SEQ ID NO:10);

a probe that comprises an amino acid sequence that: (a) is a selectively mutated TSE sequence; (b) is destabilized and non-infectious; and (c) has an amino acid sequence that is homologous to amino acid sequences 104-122 or wild-type (wt) TSE (human Nref 00130350)
KPKTNLKHVAGAAAAGAVV (SEQ ID NO:10);

a probe that comprises an amino acid sequence that: (a) is a selectively mutated TSE sequence; (b) is destabilized and non-infectious; and (c) has an amino acid sequence that is equivalent to amino acid sequences 104-122 or wild-type (wt) TSE (human Nref 00130350)
KPKTNLKHVAGAAAAGAVV (SEQ ID NO:10);

a probe that comprises an amino acid sequence that: (a) is a selectively mutated TSE sequence; (b) is destabilized and non-infectious; and (c) has an amino acid sequence that is between about 70% and about 90% identical to amino acid sequences 104-122 or wild-type (wt) TSE (human Nref 00130350)
KPKTNLKHVAGAAAAGAVV (SEQ ID NO:10);

a probe comprising amino acid sequences that are identical to amino acids 1-38 of the human islet amyloid polypeptide precursor (amylin) protein (Accession # NP_000406; human) implicated in human diabetes
MGILKLQVFLIVLSVALNHLKATPIESHQVEKRKCNTA (SEQ ID NO:11);

a probe comprising amino acid sequences that are identical to at least 10 contiguous amino acid residues within the sequence corresponding to amino acids 1-38 of the human islet amyloid polypeptide precursor (amylin) protein (Accession # NP_000406; human) implicated in human diabetes
MGILKLQVFLIVLSVALNHLKATPIESHQVEKRKCNTA (SEQ ID NO:11);

a probe comprising amino acid sequences that are identical to amino acids 1-25 of the human lung surfactant protein (NCBI Accession # AAH32785; human) implicated in human infant SIDS
MAESHLLQWLLLLLPTLCGPGTAAW (SEQ ID NO:12);

a probe comprising amino acid sequences that include at lease 10 contiguous amino acid residues of amino acids 104-122 of the human PrP^{Sc} or amino acids 103-121 of the murine PrP^{Sc} protein (SEQ ID NO:13 and 14) (SwissProt P04156; Pfam ID prion PF00377 & 03991)

Human prion protein (accession P04156)





 (SEQ ID
NO:13);

Mouse prion protein (accession PO4925)

MANLGYWLLALFVTMWTDVGLCKKRPKPGGWNTGGSRYPGQGSPGGNRYPPQ
GGTWGQPHGGGWGQPHGGSWGQPHGGSWGQPHGGGWGQGGGTHNQWNKPS
KPKTNLKHVAGAAAAGAVVGGLGGYMLGSAMSRPMIHFGNDWEDRYYRENM
YRYPNQVYYRPVDQYSNQNNFVHDCVNITIKQHTVTTTTKGENFTETDVKMME
RVVEQMCVTQYQKESQAYYDGRRSSSTVLFSSPPVILLISFLIFLIVG (SEQ ID
NO:14);

a probe comprising amino acid sequences which include at least 10 contiguous amino acid residues of amino acids 235-269 (emphasized below by double underlining) of the human plasma gelsolin (P06396; Muary *et al., FEBS Lett.* **260**(1):85-87, 1990):

MAPHRPAPALLCALSLALCALSLPVRAATASRGASQAGAPQGRVPEARPNSMVV
EHPEFLKAGKEPGLQIWRVEKFDLVPVPTNLYGDFFTGDAYVILKTVQLRNGNLQ
YDLHYWLGNECSQDESGAAAIFTVQLDDYLNGRAVQHREVQGFESATFLGYFKS
GLKYKKGGVASGFKHVVPNEVVVQRLFQVKGRRVVRATEVPVSWESFNNGDCF
ILDLGNNIHQWCGSNSNRYERLKATQVSKGIRDNERSGRARVHVSEEGTEPEAM

LQVLGPKPALPAGTEDTAKEDAANRKLAKLYKVSNGAGTMSVSLVADENPFAQ
GALKSEDCFILDHGKDGKIFVWKGKQANTEERKAALKTASDFITKMDYPKQTQV
SVLPEGGETPLFKQFFKNWRDPDQTDGLGLSYLSSHIANVERVPFDAATLHTSTA
MAAQHGMDDDGTGQKQIWRIEGSNKVPVDPATYGQFYGGDSYIILYNYRHGGR
QGQIIYNWQGAQSTQDEVAASAILTAQLDEELGGTPVQSRVVQGKEPAHLMSLF
GGKPMIIYKGGTSREGGQTAPASTRLFQVRANSAGATRAVEVLPKAGALNSNDA
FVLKTPSAAYLWVGTGASEAEKTGAQELLRVLRAQPVQVAEGSEPDGFWEALG
GKAAYRTSPRLKDKKMDAHPPRLFACSNKIGRFVIEEVPGELMQEDLATDDVML
LDTWDQVFVWVGKDSQEEEKTEALTSAKRYIETDPANRDRRTPITVVKQGFEPPS
FVGWFLGWDDDYWSVDPLDRAMAELAAYERLKATQVSKGIRDNERSGRARVH
VSEEGTEPEAM (SEQ ID NO:16);

a probe comprising amino acid sequences that include at least 10 contiguous amino acid residues of the amyloid forming region (amino acids 26-147; emphasized by double underlining below) of the cystatin C protein sequence, as depicted below and reported by Levy et al., J. Exp. Med. 169(5):1771-1778, 1989 (P01034). An appropriate probe is any portion thereof of at least 10 amino acids. Numerous probes can be positioned accordingly.

MAGPLRAPLLLLAILAVALAVSPAAGSSPGKPPRLVGGPMDASVEEEGVRRALDF
AVGEYNKASNDMYHSRALQVVRARKQIVAGVNYFLDVELGRTTCTKTQPNLDN
CPFHDQPHLKRKAFCSFQIYAVPWQGTMTLSKSTCQDA (SEQ ID NO:17);

a palindromic probe of the cystatin C protein taken from amino acids 39-47 of the above sequence, with a four unit proline linker:

### EEEVSADMPPPPMDASVEEE (SEQ ID NO:18)

a probe comprising amino acid sequences that include between 10 and 23, inclusive, contiguous glutamine resides of oligo or polyglutamine from residues 18-40 (emphasized by double underlining below) of the Huntingtin protein (Huntington's disease protein) protein sequence depicted below:

MATLEKLMKAFESLKSFQQQQQQQQQQQQQQQQQQQQQQQPPPPPPPPPPPQLP
QPPPQAQPLLPQPQPPPPPPPPPPGPAVAEEPLHRPKKELSATKKDRVNHCLTICEN
IVAQSVRNSPEFQKLLGIAMELFLLCSDDAESDVRMVADECLNKVIKALMDSNLP
RLQLELYKEIKKNGAPRSLRAALWRFAELAHLVRPQKCRPYLVNLLPCLTRTSKR
PEESVQETLAAAVPKIMASFGNFANDNEIKVLLKAFIANLKSSSPTIRRTAAGSAV
SICQHSRRTQYFYSWLLNVLLGLLVPVEDEHSTLLILG (SEQ ID NO:19; P42858;
gi:1170192);

an exemplary probe: QQQQQQQQQQQQQQQQQ (SEQ ID NO:20);

a probe comprising amino acid sequences that include at least 6 contiguous amino acid residues of amino acid residues 45-50 and 48-53 (emphasized below) of the human islet amyloid polypeptide involved in fibrillogenesis, sequence depicted below (SEQ ID NO:21) NP_000406 [gi:4557655] Scrocchi et al., J. Struct. Biol. 141(3):218-227, 2003.

MGILKLQVFLIVLSVALNHLKATPIESHQVEKRKCNTATCATQRLANFLVHSSNN
FGAILSSTNVGSNTYGKRNAVEVLKREPLNYLPL (SEQ ID NO:21).

Exemplary probes contain the following sequences, which are minimal sequences within the sequence 45-53 of the above peptide sequence of SEQ ID NO:21, which may be used without modification or may be used to form palindromic probes of the present invention:
LANFV (SEQ ID NO:22);
VFNALPPPPLANFV (palindromic probe) (SEQ ID NO:23);
FLVHSS (SEQ ID NO:24);
SSHVLFPPPPFLVHSS (palindromic probe) (SEQ ID NO:25);

a probe comprising amino acid sequences that include at least 5 contiguous amino acid resides of amino acid residues 11-19 (emphasized below by double underlining) of the peptide fragment of transthyretin (AAH20791 [gi:18089145]; MacPhee and Dobson, J. Mol. Biol., 279(5):1203-1215, 2000)



 (SEQ ID NO:26);

a palindromic probe based on the above-referenced emphasized sequence of SEQ ID NO:26 (amino acid residues 11-19):
ESVFVLGALPPPPLAGLVFVSE (SEQ ID NO:27).

Numerous other probes may be readily produced without undue experimentation using standard laboratory techniques and peptide a related chemical syntheses.

The native conformation of the probe is determined by one or more spectroscopic methods, such as CD, Fourier transform infra-red, ultra-violet, NMR, or fluorescence, among others. This conformation in a solvent as described below should correspond to that of an alpha-helix or random coil (in CD, for example, the nature of the spectrum is indicative of the conformation).

The probe is modified to contain substituents that are detectable by optical means. Such substituents may include tryptophan (an amino acid), pyrene or similar fluorophores, all attached at or near the terminal positions of the peptide probes. Attachment of such fluorophores proceeds according to conventional chemical methods, which are well known in the art. Preferably, but not necessarily, attachment is through covalent attachment of the fluorophor to the probe. Ideally, the substituents have the capability to interact in such a manner as to produce a species known as an excimer. An excimer represents the interaction of two fluorophores that, upon excitation with light of a specific wavelength, emits light at a different wavelength, which is also different in magnitude from that emitted by either fluorophor acting alone. Thus, structural alterations of the conformational probe that allow for the formation of such excimers can be detected by a change in optical properties. Such changes can be measured by known fluorimetric techniques, including UV, IR, CD, NMR, or fluorescence, among numerous others, depending upon the fluorophor attached to the probe. The magnitude of these changes is related to the degree to which the probe has undergone the conformational alteration.

In another embodiment, the probe may be substituted with a radioactive material. Ideally, this should be positron emission of a sufficient energy to be detected by machines currently employed for this purpose. Such an entity would preferably contain oxygen-15 (an isotope of oxygen that decays by positron emission) or other radionuclide. In this embodiment, the radiolabeled probe may be injected into a patient and the binding of the probe to the protein target monitored externally.

A probe may comprise a peptide or peptidomimetic of at least five, preferably about ten or more, amino acid residues that exhibit a random coil or alpha-helical conformation in solution. A peptide or peptidomimetic probe solvent may be aqueous and have a pH of between about 4 and about 10, preferably between about 5 and about 8, and may have an ionic strength of between about 0.05 and about 0.5 (when typically prepared with a chloride salt, such as sodium chloride or potassium chloride). The solvent may also contain a percentage of a water-miscible organic material, such as trifluoroethanol in amounts between about 30% to about 70% by volume, preferably between about 45% to about 60%. The solvent may be prepared with a suitable buffering system such as acetate/acetic acid, Tris, or phosphate.

The sequence of probe amino acids is determined from the nature of the target protein to be analyzed and usually comprises a region of the target that is known to undergo a structural transition from either an alpha-helix or coil to a beta-sheet. This latter structure is associated with the pathogenic form of the target protein. The conformational probe sequence ideally contains two repeats of the target sequence of interest, preferably between about 10 and 25 amino acids in length, more preferably between about 14 and 20 amino acids in length. These are arranged preferably in the probe to form a palindrome as illustrated in Figure 10.

Preferred probes used in methods and kits of the invention have amino acid sequences corresponding to β-sheet regions of the protein to be analyzed. These probes are preferably at least 5 amino acids units in length and can be about 300-400 amino acid units in length (-mer) or more, although, preferably these are about 10 amino acids to about 50 amino acids in length. In certain aspects of the invention, preferred probes that correspond to the β-sheet region are about 15 amino acids in length to about 100 amino acids in length. In others, preferred probes may range from about 20 amino acids in length to about 40 amino acids in length. The preferred length of a given probe will be a function of the probe's ability to complex and produce β-sheet formation with the target protein.

Probes for use in the present invention are readily determined from existing information in sequence databases or, alternatively, may be readily determined experimentally. Thus, the probe will generally correspond to a minimum number of amino acids, preferably at least 10, and more preferably about 10 to 25 amino acids, which correspond to at least a portion of a peptide sequence of a target protein that can undergo a conformational transition from alpha-helix or random coil to β-sheet formation in the insoluble protein.

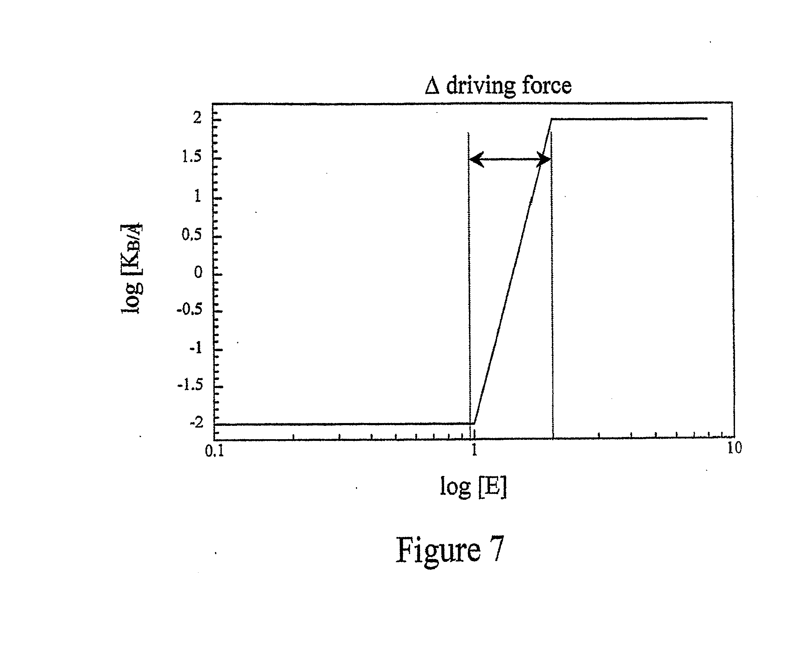

Noting that within the experimental information that will guide the presentation and synthesis of an appropriate probe, there are some constraints that can guide the practitioner in making use of the present invention. There are only a few kcal difference separating a population in the initial conformation state from a population predominantly in the transformed conformational state (in complex). As depicted in Figure 7, the transformation from one conformational state to the other is provided by the driving force due either to the Kd of association between the probe molecule and its natural associate to form β-sheet complex, or to changes in the electrostatic interactions between the molecules (for example, changes caused by lowering the ionic strength of the solution). If metal ions, such as Al, or the binding of another ligand are involved, other electrostatic or stearic effects could contribute. The size of the probe peptide can vary, but should be of sufficient length to have "reasonably" well defined secondary structure under detection conditions and to have sufficient recogntional specificity for the target selected, such as a prion protein. The probe peptide should also accommodate single-site mutations in order to be generally applicable to mutated proteins or strains, recognizing that these changes and/or heterogeneities affect the thermodynamic stability of the molecule. Moreover, the probe must be non-contagious to the patient population, whether that population is a human patient population, a domesticated animal population, or other mammalian population.

Once a peptide sequence is established for a probe (which corresponds to at least a portion of a target protein responsible for β-sheet formation as described above), the peptide sequence may be endcapped (at one, and preferably both, ends of the peptide) with a moiety or chemical entity that can facilitate analysis of the peptide probe. Preferably, this moiety is a fluorophor, such as pyrene, but may vary widely, depending upon the analytical technique to be used for analysis. The moiety or chemical entity may be complexed or covalently bonded at or near the amino or carboxy end of the peptide, which is preferably endcapped with a short, hydrophobic peptide sequence. In preferred aspects of the present invention, both the amino and carboxy ends of the probe peptides are endcapped with small hydrophobic peptides ranging in size from about 1 to about 5 amino acids. These may be natural or synthetic, but are preferably neutral (*i.e.,* derived from a β-sheet formation region of a target protein). The fluorophores are preferably attached at or near the amino and or carboxy end of the probe (preferably both) and may be, for example, pyrene, tryptophan, fluorescein, rhodamine, among numerous others, and is preferably pyrene. It is preferable that the fluorophores form excimers when in the correct geometric orientation.

Conformational probes according to the present invention are preferably palindromic in nature. The term palindromic refers to the organization of a given conformational probe sequence such that it will contain first and second peptide sequences corresponding to a portion of the target protein responsible for the β-sheet formation, but which peptide sequences are presented in a palindromic manner, i.e. from the carboxy end to the amino end (or amino end to carboxy end) in the first peptide sequence, and from the amino end to the carboxy end (or carboxy end to amino end) in the second peptide sequence. The first and second peptide sequences in the palindromic conformational probe do not have to be identical in length, although this may be preferred in certain embodiments, but should be at least roughly equivalent (the two peptide sequences ("arms" of the probe) should not be more than 15, preferably no more than 10, and even more preferably no more than 5 amino acids in length). Preferably, the first and second peptide sequences within a palindromic probe sequence are separated by a linker comprising between 1 and 5 amino acids, preferably between 1 and 3 amino acids, which preferably contain at least one proline amino acid and more preferably comprise primarily proline amino acids. Figure 10 presents an exemplary palindromic 33-mer conformation probe useful in the present invention.

Preferably, conformational probes according to the present invention contain a hydrophobic amino acid sequence that is preferably derived from the relevant peptide sequence of the target protein (*i.e.,* the peptide sequence responsible for β-sheet formation), and that may vary in length from 1 amino acid to 20 or more amino acids, preferably about 2-10 amino acids in length and appears at or near one of the two ends of the conformation probe. In the case of palindromic conformation probes, these hydrophobic amino acid sequences appear at the ends of the two peptide arms of the probe. Optionally, the probe also may contain a synthetic hydrophobic amino acid sequence (*i.e*., not natural to the peptide sequence of the target protein responsible for β-sheet formation) at at least one end of the probe and, in the case of palindromic probes, at or near each end of the probe, which may vary in length from as few as one amino acid to 20 or more amino acids, preferably about 3-10 amino acids in length.

By way of example and without limitation, if a desired peptide sequence in a target protein contains the sequence, reading from amino end to carboxy end, QRSTVVARLKAAAV (SEQ ID NO:15) (where AAAV (SEQ ID NO:30) is a hydrophobic amino acid sequence) then the palindrome would contain a first peptide sequence which is VAAAKLRAVVTSRQ (SEQ ID NO:31) and a second peptide sequence which is QRSTVVARLKAAAV (SEQ ID NO:15) (or a close variation to that sequence), with the two sequences separated by a linker comprising from 1 to 5 amino acids, with at least one of those amino acids, and preferably most, if not all, of those amino acids, being proline amino acids. The probe would therefore be: VAAAKLRAVVTSRQPPPPQRSTVVARLKAAAV (SEQ ID NO:28) (hypothetical palindromic probe).

Preferably, the palindromic probe would contain a hydrophobic amino acid sequence obtained from the relevant sequence of the target protein. Conformational probes according to the present invention may be readily obtained.

The following rules may be used to guide the formation of an appropriate preferred conformational probe according to the present invention. These rules apply generally to conformational probes according to the present invention without limitation, but are more specifically used in context to produce the preferred palindromic conformational probes according to the present invention.

The following rules may be applied to the instant invention to produce preferred conformational peptide probes:

1. Each "arm" of the peptide palindrome should have a minimum of five, and preferably at least 10-12 amino acids and, ideally, not more than about 25 amino acids.

2. The amino acid sequence is selected from a region of a larger protein that is known to undergo a conformational transition from alpha-helix or random coil to beta sheet.

3. One or more of the following additional criteria:

a) A high proportion of hydrophobic amino acids - generally not less than about 75% (based upon the number of amino acids), ideally 80% or greater,

b) Amino acid repeats of at least 20 and preferably 25 (such as is present in huntingtin),

c) Clustered charges of opposite sign (as described in Zhang, S., Altman, M. and Rich, A. in Conformational Disease, A Compendium, Solomon, Taraboulos and Katchalski-Katzir, eds. The Center for the Study of Emerging Diseases, 2001,

d) A linker sequence between each of the peptide arms that has 1 or more amino acids, preferably less than five and that contains one or more proline residues.

Test criteria for peptide probe:

### 1. The conformation of the palindrome peptide probe should be that of an alpha helix or random coil but not a beta sheet.

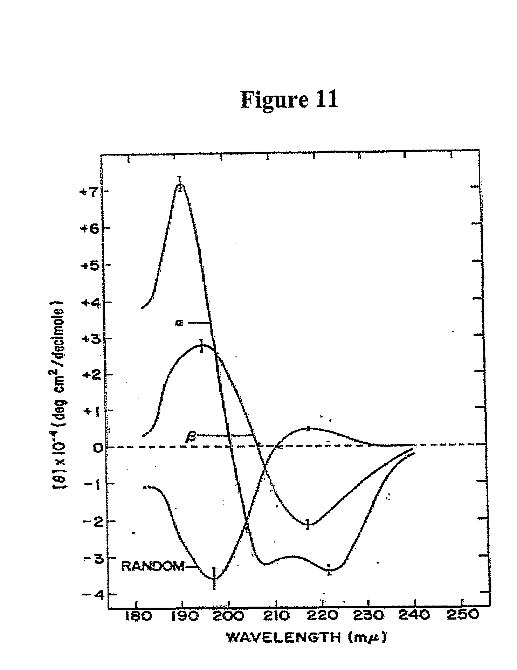

2. Determination of the conformation of the peptide is ideally accomplished by CD measurements that can identify solution conformations. These are performed using a CD spectrometer in one or more solvents that can include aqueous buffers and/or organic agents, such as trifluoroethanol - see Figure 11.

Applying the general rules obtained above and using readily available methods in the art, one of ordinary skill can produce large numbers of conformational peptide probes having favorable characteristics to be useful in the present invention.

"Circular dichroism" ("CD") is observed when optically active matter absorbs L and R hand circular polarized light slightly differently, as measured by a CD spectropolarimeter. Differences are very small and represent fractions of degrees in ellipticity. Figure 11 depicts an associative CD curve representative of the three distinct common conformational forms that proteins and peptides can assume. CD spectra for distinct types of secondary structure present in peptides and proteins are distinct. Measuring and comparing CD curves of complexed vs, uncomplexed protein represents an accurate measuring means of practicing the present invention.

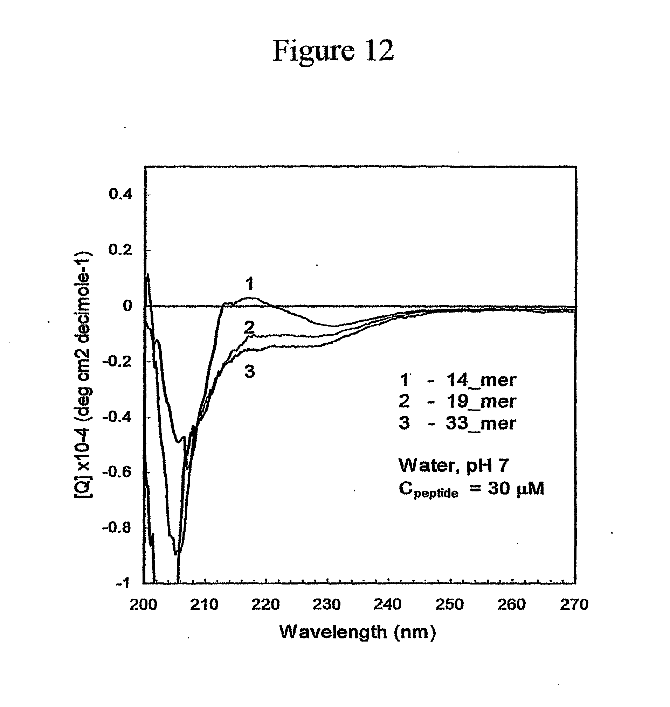

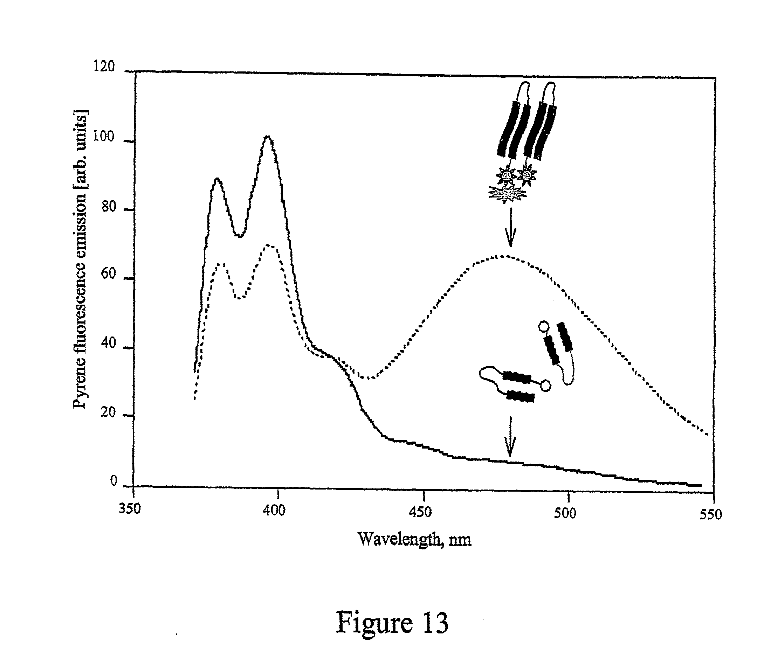

Unexpectedly, we have determined that under near physiological conditions, the palindrome 33-mer (SEQ ID NO:1 or 29), which covalently connects two peptides - in the case of the human palindromic probe, the sequence depicted in Figure 10, whereas in the case of a murine probe, a 14-mer (SEQ ID NO:3) and a 19-mer (SEQ ID NO:2) - exhibits a largely random coil or α-helix conformation despite the proximity of two hydrophobic chains resembling the 14-mer structure, as illustrated in Figure 12. The addition of a pyrene at each end of the palindromic 33-mer peptide allows for spectral observation of the conformational change, as illustrated in Figure 13. The spectral scans for pyrene attached to the ends of the 33-mer in the monomer (open) conformation gives a strikingly different fluorescent spectrum, having a maximum emission between 370 and 385 nm, while the excited dimer or excimer state of the pyrene-labeled peptide has an emission maximum between 475 and 510 nm.

Although it is possible to follow conformational changes by any of the several optical methods described above, a preferred embodiment of the invention utilizes fluorescence spectroscopy because that techniques provides sensitivity, rapidity, and simplicity of operation. The probe is modified by attachment at both termini of a fluorophor that has specific optical properties. It is preferred that these include the ability to fluoresce upon irradiation with light of a specific wavelength (defined by the absorption and emission spectra of the fluorophor itself). Thus, probes are preferably designed such that irradiation with light of a wavelength near that of the absorption maximum causes the probe to emit light at a sufficiently higher wavelength so as to be distinguished from the excitation wavelength. Measuring such irradiation and emission, and techniques for doing so, are well known to those versed in the art. Examples of such fluorophores include, but are not limited to, pyrene, tryptophan, fluorescein, and rhodamine. It is also preferred that the attached fluorophores have the capacity to form excimers when in the correct geometric orientation.

An "excimer" is an adduct that is not necessarily covalent and that is formed between a molecular entity that has been excited by a photon and an identical unexcited molecular entity. The adduct is transient in nature and exists until it fluoresces by emission of a photon. It is possible to recognize an excimer (or the formation of an excimer) by the production of a new fluorescent band at a wavelength that is longer than that of the usual emission spectrum. An excimer can be distinguished from fluorescence resonance energy transfer since the excitation spectrum is identical to that of the monomer.

The formation of the excimer is dependent on the geometric alignment of the fluorophores and is heavily influenced by the distance between them. In a preferred embodiment, fluorophores are present at each probe terminus and excimer formation between fluorophores is negligible as long as the overall probe conformation is alpha-helix or random coil. This is readily determined by measurement of the fluorescent behavior of the probe in the solvent to be used for analysis in the absence of the target protein to be measured.

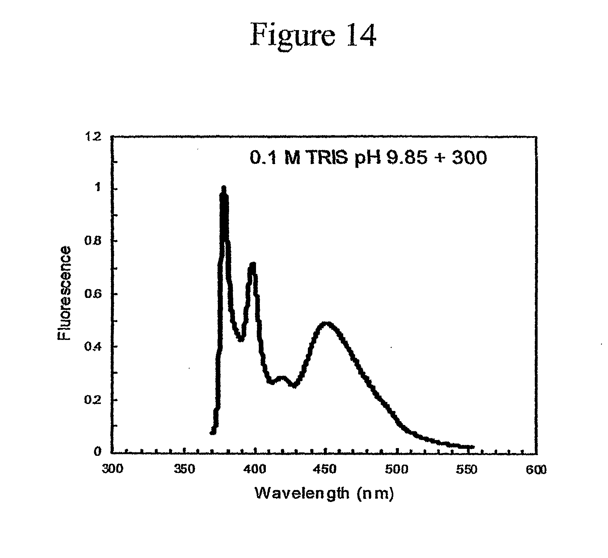

Preferred conformational transition following interaction with an analyte target is achieved by measuring fluorescence spectra under conditions where excimer formation can be analyzed. Typically, using pyrene as an exemplary fluorophor, the excitation wavelength would be about 350 nm and the observation wavelength 365-600 nm. The normal emission of monomer pyrene following excitation (simple fluorescence) is recorded as the maximum wavelength between about 370-385 nm. Representative data is shown in Figure 14.

As shown in Figure 14, the excimer or excited dimer state is recorded at a maximum of between 475-510 nm. The formation of the excited dimer state can also be encouraged through the addition of high salt and by conducting measurements at a pH approaching the pI of the peptide (*e.g*., in the illustrated case, a pH of around 10).

Therefore, in a preferred method of the invention, interaction of the probe with the specific protein to be analyzed causes a conformational change in the probe such that excimer formation occurs. This is readily measured by the procedures described herein. Conversion of the probe structure from that exhibited in the absence of analyte (alpha-helix or random coil) to a beta-sheet structure enables fluorophores attached to the probe to form excimers that can be readily identified. Further, the magnitude of excimer formation is directly related to the amount of protein analyte present.

Proteins or prions may be detected in aggregated form or in the presence of other cellular constituents, such as lipids, other proteins, or carbohydrates. A sample preparation for analysis is preferably homogenized or subjected to a similar disruption of tissue or aggregate structures, and cellular debris is preferably removed by centrifugation. This process is ideally performed in the presence of a buffered salt solution and may utilize one of several detergents such as SDS, Triton X-100, or sarkosyl. Further concentration of the sample may be achieved by treatment with any of several agents; one preferred agent is phosphotungstate, which is employed according to the method of Safar et al., Nature Medicine 4:1157-1165, 1998.

In a preferred embodiment of the invention, peptide probes are selected for addition to an unknown or test sample. The peptide probes are preferably proteins or peptide sequences that have secondary structures of predominately alpha-helix or random coil, but which are preferably, but not necessarily, derived from portions of a target peptide responsible for β-sheet formation. In a particularly preferred embodiment, the peptide probes are peptide fragments consisting of a helix-loop-helix structure found in polylysine. In another particularly preferred embodiment, the peptide probes can be made of a peptide sequence chosen from wild-type TSE, from a desired species-specific TSE peptide sequence, or even from a selectively mutated TSE sequence that has been mutated in such a manner as to render it destabilized and non-infectious. Additionally, extrinsic fluors, such as pyrene, can be added or designed into the peptide probe to allow detection of anticipated conformational changes using common fluorescence detection techniques.

Once a peptide is selected, it is added to a test sample. Prior to the addition of the peptide probe, however, it is preferred to have the sample subjected to disaggregation techniques commonly known in the art, such as sonication. The disaggregation step allows any potentially aggregated sample material to break apart so that these disaggregated sample materials are free to combine with the newly introduced peptide probe, thereby facilitating the anticipated catalytic propagation.

After the test sample or disaggregated test sample is allowed to interact with the peptide probes, the resulting mixture is then subjected to analytical methods commonly known in the art for the detection of aggregates and to fluorescence measurements in cases where fluorescent peptide probes are used. Unknown or test samples containing any dominant beta-sheet formation characteristic of abnormally folded or disease-causing proteins result in an increase in beta-sheet formation and consequently aggregate formation in the final mixture containing both the test sample and the peptide probes. Conversely, unknown or test samples that lack any predominantly beta-sheet secondary structures will neither catalyze a transition to beta-sheet structure nor will propagate the formation of aggregates.

The initial conformational change can be triggered in the test samples in a number of ways. Without intending to be bound by any theory, the binding of a metal ligand could direct a change in the protein conformation and favor aggregation. The expression or cleavage of different peptide sequences can promote advanced aggregation leading to fibril and plaque formation. Genetic point mutations can also alter the relative energy levels required of the two distinct conformations, resulting in midpoint shifts in structural transitions. Furthermore, an increase in concentration levels could be sufficient to favor the conformational transition. Regardless of the initial trigger mechanism, however, the disease process in many of the abnormal protein conformations, such as in prion-related diseases, involves the catalytic propagation of the abnormal conformation, resulting in structural transformation of the previously normal protein.

Optical detection techniques useful in the present invention include, but are not limited to, light scattering, hydrophobicity detection using extrinsic fluors, such as 1-anilino-8-naphthalene sulfonate (ANS) or Congo Red stain, fluorescence resonance energy transfer (FRET), quenching of intrinsic tryptophan fluorescence through either conformational change or monomer or binding at an interface in an alpha-beta heterodimer, equilibrium ultracentrifugation, and size-exclusion chromatography,

It has unexpectedly been determined that immobilizing the peptide probes of the invention on a solid support, such as a polystyrene plate, can "lock" the probes into a particular conformation (*i.e.,* cause the probes to maintain a predominantly α-helical form or a predominantly β-sheet form). While immobilization of various peptides to various solid supports is known in the art, typically, one cannot predict with a reasonable degree of assurance that any particular peptide can be immobilized with satisfactory efficiency. Furthermore, one cannot predict with a reasonable degree of assurance that a peptide, once immobilized, will have the desired activity (*e.g*., retain the activity it shows in solution). Indeed, it is well known in the art that immobilization of a peptide can result in denaturation of the peptide, and thus destruction of most, if not all, of its secondary structures. Thus, it is well known that only through careful selection of conditions, often through trial-and-error experimentation, can one identify the proper conditions for immobilization of a particular peptide, and it is not generally possible to predict the conditions that will successfully immobilize a given peptide while retaining its secondary structures and biological activity. The present invention discloses the unexpected result that probes according to the invention, which, as discussed above, share common structural characteristics, can not only be immobilized onto solid supports with adequate efficiency, but can also retain their activity once immobilized. Furthermore, the present invention discloses that probes that are immobilized in a particular conformation (*e.g*., β-sheet or α-helix) can retain the activity associated with that conformation, even when exposed to conditions that would alter their conformation if they were in solution. Thus, the present invention addresses and solves a problem confronting the art: how to provide an immobilized probe or set of probes that specifically binds to a prion protein of interest.

The discoveries that the probes of the inventions can be "locked" into a particular conformation by immobilization, and that the conformations are active in binding prion proteins, permits one practicing this invention to use the probes of the invention in numerous applications. For example, one can use probes immobilized to beads to rapidly and efficiently detect the presence of prion proteins in a sample (*e.g*., by using an immobilized α-helical probe) or to preferentially detect infective prion proteins in a sample (*e.g*., by using an immobilized β-sheet probe). One can also use immobilized probes to bind some, essentially all, or all prion proteins present in a sample, then separate the prion proteins from the rest of the sample, to provide a purified sample that has fewer prion proteins, is essentially prion free, or is completely prion-free. These techniques have implications for the health care field, particularly those aspects involved in production and use of blood products. For example, this invention can be used not only to detect the presence of prions in a blood or blood product sample, but to reduce the number of, or completely eliminate, prions in the blood or blood product sample.

Thus, in embodiments of the invention, methods of immobilizing probes of the invention are provided. The methods generally comprise providing a probe of the invention, which can include creating a composition comprising the probe in a desired conformation; providing a solid support, exposing the probe to the solid support for a sufficient amount of time to permit immobilization of the probe to the solid support, and removing unbound probe and other substances present in the composition. The method can further comprise cross-linking the probe to the solid support, washing the bound probe to remove unbound probe or other substances, drying the solid support and probe, or other procedures that are known to be used in creating protein-solid support combinations. Of course, prior to binding of the probe to the solid support, the probe can be exposed to a misfolded protein, such as a mis-folded prion protein (PrP^{Sc}), for a sufficient amount of time for the probe to assume a predominantly β-sheet conformation and be bound to the mis-folded protein. Binding of the probe-misfolded protein complex to the solid support then proceeds as usual. In such a situation, the probe-misfolded protein complex can be bound to the solid support directly, via the probe, or indirectly via binding of the misfolded protein to another probe (either a probe of the present invention or another probe, such as one known in the art) that is bound to the solid support. Furthermore, the probe and/or the probe-misfolded protein complex can be purified to any suitable extent prior to or after binding with each other or with the solid support.

In creating a composition comprising the probe in a desired conformation, one can use different pH or salt compositions to affect the conformation of the probes of the invention. As discussed above, the conformations of the probes of the invention can be altered by adjusting the salt concentration, the pH, or both, of the solution in which the probes are present. In general, conditions that approach the pI of each probe cause the probe to assume a predominantly β-sheet conformation. Likewise, acidic (*e.g.,* pH less than about 5.0) or basic (*e.g.*, pH more than about 9.0) conditions cause the probe to assume a predominantly β-sheet conformation. In contrast, neutral (*e.g*., pH between about 5.0 and about 9.0) conditions cause the probe to assume a predominantly α-helix structure. In general, low salt concentrations (*e.g*., below 100 mM salt) cause the probe to assume an α-helix conformation, whereas high salt concentrations (*e.g.,* 500 mM salt) causes the probe to assume a β-sheet conformation. One of skill in the art can select a suitable combination of pH and salt concentration to obtain probes of the desired conformation without undue experimentation.

The solid support can be any known solid substance that is suitable for binding peptides and use with biological materials. Many such solid supports are known to those of skill in the art. Examples of materials that are useful as solid supports, include, but are not limited to, plastics, including polystyrene, glass, polysaccharides, metal, and various polymers, including latex, acrylics, and nylons. Examples of forms of solid supports include, but are not limited to, plates, beads, and membranes. Many types of modifications to solid supports are known to enhance binding of peptides to them, and all such modifications and modified solid supports are encompassed by the present invention. Included among the solid supports are those with intrinsic optical properties, such as one in which the optical properties change upon binding of a substance to it, and one in which the optical properties change upon binding of a substance to a peptide bound to the support. For example, a solid support can be one that has intrinsic fluorescence and can bind a probe according to the invention, where, upon binding of a protein to the probe, the fluorescent properties of the solid support changes, facilitating detection of binding.

The amount of time the probe will be exposed to the solid support will vary depending on the type of solid support used, the amount of probe supplied, and other variables that one may select. In general, the amount of time will be the amount that is typically used to bind peptides to the solid support, and typically be based on the solid support and the suggestions provided by the solid support manufacturer. Thus, the amount of time can be as short as one minute, or as long as one week. In general, exposure will comprise from about one hour to about 72 hours, such as about 2 hours, about 3 hours, about 14 hours, about 16 hours, about 24 hours, or about 48 hours. Binding efficiency can be monitored in test runs to identify the optimal binding time and conditions.

Removing unbound probe and other substances present in the composition can be accomplished by any known suitable technique. Typically, it comprises removing the solid support-bound peptide combination from the binding composition, or removing the binding composition from the solid support-peptide combination. Thus, removing can be accomplished by pouring the composition out from the container in which it and the solid support were exposed to each other. It can likewise be accomplished by sucking, aspirating, siphoning, draining, or the like. In a particular embodiment, removing is accomplished by permitting the composition to evaporate.

The method can further comprise other steps that are known to be useful in creating solid support-bound peptide combinations. As is known in the art, many solid supports that are available for binding of peptides have surfaces that are designed to covalently bind peptides through cross-linking of the probe to reactive groups on the surface of the solid support. Such cross-linking can be effected in numerous ways, including irradiation with ultraviolet light, treatment with heat or chemicals, or drying. Any suitable solid support and technique is encompassed by this invention.

Other additional steps can include washing the bound probe to remove unbound probe or other substances that are present in the composition, drying the solid support and probe, or other procedures that are known to be used in creating protein-solid support combinations. One may choose from among the various additional treatments and combinations of treatments to suit a particular need.

The immobilized probes have numerous uses. In general, the immobilized probes are used to bind to prion proteins in a sample. Binding can be used for many purposes, such as to detect the presence of prion proteins in the sample, or to bind prion proteins in a sample and remove them. Detecting prion proteins in a sample can be used in a method of screening products, such as food products or medical products (*e.g.,* blood products) for contamination with prion proteins, including infective prion proteins such as PrP^{Sc}. Removal of prion proteins from a sample can be used in a method of reducing or eliminating prion proteins from products, such as food products or medical products (*e.g*., blood products), which can improve the safety of the products for human or animal use.

In general, a method of screening using an immobilized probe comprises providing an immobilized probe, providing a sample containing or suspected of containing a prion protein, exposing the sample to the immobilized probe under conditions and for an amount of time sufficient for the immobilized probe to bind to a prion protein in the sample (if present), and detecting the presence of prion protein bound to the immobilized probe. Detection can be by way of any known technique, as discussed and detailed above. In embodiments, detection comprises assaying emission of light from a label, such as by fluorescence or luminescence. In other embodiments, detection is by PAGE and staining of proteins present in the gel. In yet other embodiments, detection is by reaction with an antibody specific for a prion protein of interest. Other non-limiting examples of detection techniques are given above.

Exposing the sample to the immobilized probe is for a sufficient amount of time and under conditions that permit binding of a prion protein (if present in the sample) to the immobilized probe. Suitable conditions for binding of proteins to other proteins are known to those of skill in the art, and any suitable conditions can be used. Exemplary conditions are detailed in the Examples that follow. In general, suitable conditions comprise an aqueous environment, neutral pH (*e.g*., pH from about 6.0 to about 8.0), moderate salt (*e.g.,* from about 100 mM to about 400 mM salt), and little or no detergents, emulsifiers, or other ancillary substances that might inhibit protein-protein interactions. The amounts of immobilized probe and sample to be used will vary depending on the amount of sample available, the amount of prion protein suspected of being present in the sample, the amount of time the user wishes to expose the sample to the immobilized probe, and other considerations. The amount of immobilized probe and sample will, in general, be the same as the amounts used when non-immobilized probe is used, as detailed above. In embodiments, the sample contains or is suspected of containing an infective or disease-causing prion protein, such as PrP^{Sc}.

In general, a method of reducing prion protein in a sample comprises providing an immobilized probe, providing a sample containing or suspected of containing a prion protein, exposing the sample to the immobilized probe under conditions and for an amount of time sufficient for the immobilized probe to bind to at least some of the prion proteins in the sample (if present), and separating the immobilized probe and immobilized probe-prion protein complexes from the sample. In embodiments, the method reduces the amount of prion protein in the sample by an amount that is detectable. In embodiments, the method reduces the amount of prion protein in the sample to an amount below detection limits. In embodiments, the method completely eliminates prion proteins from a sample. In embodiments, the sample contains or is suspected of containing an infective or disease-causing prion protein, such as PrP^{Sc}.

In the method of reducing prion protein in a sample, the immobilized probe and sample can be the same as those discussed in the method of detecting a prion protein, and can be provided in the same amounts and forms as discussed above. Separating the immobilized probe and immobilized probe-prion protein complexes from the sample can be by any suitable technique, such as by pouring off of the sample, by physical removal of the immobilized probe and complexes from the sample, etc. Those of skill in the art are aware of numerous ways of removing beads, membranes, and other solid supports from aqueous solutions, and any of those ways can be used to separate the immobilized probe and immobilized complexes from the sample. In a preferred embodiment, the immobilized probe is a probe bound to a membrane that is permeable to blood or blood products, such as plasma, the sample is blood or a blood product, such as plasma, and the blood or blood product is filtered through the probe-bound membrane to eliminate some or all of the prion proteins in the blood or blood product. Passing of the last of the sample across the membrane causes separation of the probe-bound membrane and the sample. After the blood or blood product has been filtered, the probe-bound membrane can be assayed for the presence of prion proteins.

As is evident from the above disclosure, in embodiments, the method further comprises detecting the presence of prion protein bound to the immobilized probe. Detection can be by way of any known technique, as discussed and detailed above. Likewise, various additional steps can be included in the methods, as long as those steps do not render the methods incapable of removing some or all of the prion proteins present in a sample.

In any of the methods of this invention, controls (either positive, negative, or both) can be run to validate the assay. Positive controls generally comprise performing the methods with samples that are known to comprise at least one prion protein (typically of a specific, known type), and can be used to confirm that the methods are capable of detecting that protein and/or are specific for that particular protein. Generally, a positive control comprises a sample (at any stage of the procedure) to which is intentionally added a known prion protein, typically in a known amount. Negative controls generally comprise performing the methods with samples that are known not to contain any prion proteins, and can be used to confirm that the methods are not providing systematic false positive results. Other controls can be run at one or more particular stages in the methods to verify that those stages are functioning as expected. One of skill in the art is well aware of suitable controls and can design and implement them without undue experimentation.

As discussed in detail in the Examples below, it has been found that probes of the invention that are immobilized in a predominantly β-sheet conformation are specific for disease-causing forms ofprion proteins (*e.g*. PrP^{Sc}). Thus, immobilization of probes can be used to design and implement methods of specifically detecting disease-causing prion proteins and methods of reducing or eliminating disease-causing prion proteins. This is an advantage over other available methods because it permits specific detection of a subset of prion proteins without the need for expensive and time-consuming use of antibodies.

Thus, in embodiments, the invention provides a method of reducing or eliminating PrP^{Sc} from a sample. The method comprises providing an immobilized probe that comprises predominantly a β-sheet conformation, contacting the immobilized probe with a sample containing or suspected of containing PrP^{Sc} under conditions and for a sufficient amount of time for the immobilized probe to bind to at least some of the PrP^{Sc} in the sample, and separating the probe/PrP^{Sc} complexes from the sample. In embodiments, the sample is a biological sample, such as blood or plasma.

In view of the methods discussed above, it is evident that the present invention provides kits comprising, in packaged combination, at least one immobilized probe having a specific conformation. More specifically, because immobilization can result in a solid support comprising a probe in a particular conformation, which remains in that conformation during subsequent exposure to various environmental conditions, the present invention can provide kits containing such immobilized probes. As discussed herein, the immobilized probes can be used to specifically bind prion proteins of choice, and thus can be used to detect specific prion proteins in a sample or can be used to remove specific prion proteins from a sample. By "packaged combination" it is meant that the kits provide a single package that contains a combination of one or more components, such as immobilized probes, buffers, instructions, and the like. Although a kit containing a single container would normally not be considered to have a combination of elements, for the purposes of this invention, such a configuration is included within the definition of "packaged combination".

In general, the kits of this aspect of the invention comprise some or all of the immobilized probes and other supplies and reagents necessary to practice a method of the invention. Thus, they typically comprise at least one immobilized probe in at least one container. In certain embodiments, a single probe (including multiple copies of the same probe) is immobilized on a single solid support and provided in a single container. In other embodiments, two or more probes, each specific for a different prion protein or a different form of a single prion protein, are provided in a single container. In embodiments, the same immobilized probe is provided in multiple different containers (*e.g*., in single-use form), or multiple immobilized probes are provided in multiple different containers. In embodiments, the probes are immobilized on multiple different types of solid supports. Any combination of immobilized probe(s) and container(s) is contemplated by the invention, and the practitioner is free to select among the combinations to achieve a suitable kit for a desired use.

A container of the kits can be any container that is suitable for packaging and/or containing the immobilized probes of the invention. Suitable materials include, but are not limited to, glass, plastic, cardboard or other paper product, and metal. The container can completely encase the immobilized probes or can simple cover the probe to minimize contamination by dust, oils, etc. The kits can comprise a single container or multiple containers, and where multiple containers are present, each container can be the same as all other containers, different than others, or different than some, but not all other containers.

The kits themselves can be made of any suitable material. Non-limiting examples of kit materials are cardboard or other paper product, plastic, glass, and metal. Cardboard kits are preferred.

Kits can comprise some or all of the reagents and supplies needed for immobilizing one or more probes to the solid support, or some or all of the reagents and supplies needed for binding of immobilized probes to prion proteins in a sample.

Of course, the kits of the invention can comprise one or more non-immobilized probe and one or more solid support that does or does not comprise an immobilized probe. Such kits preferably comprise some or all of the reagents and supplies needed for immobilizing one or more probes to the solid support, or some or all of the reagents and supplies needed for binding of immobilized probes to prion proteins in a sample.

The present invention uses propagated conformational change to correlate directly levels of abnormal proteins or prions with levels of infectivity. For this reason, it is preferable to utilize the methods of the invention in a manner in which there is no increase in infectious products as a result of the propagation. This can be achieved by placing a "break" in the links between the chain of infection, transmission, and propagation of the abnormal form. Such a break must occur at the transitional stage between the dimer and multimer forms of the aggregate. The physical formation of the multimer form can be blocked by simply impeding the step that leads to its formation. This may be achieved by using a probe in which the sequence of interest is attached to a non-relevant peptide, or by a neutral "blocker" segment, with the understanding that probes on linkers or "tethers" are more likely to encounter each other and thus result in amplifying the signal.

### EXAMPLES

The invention will be further clarified by the following examples, which are intended to be purely exemplary of the invention, and should not be considered as limiting the invention in any way.

### Example 1

This Example provides the materials and methods that are used in the other Examples, unless otherwise noted. A sample may be obtained for testing and diagnosis through use of the present invention as follows. A sample may be prepared from tissue (*e.g*., a portion of ground meat, or an amount of tissue obtained by a biopsy procedure) by homogenization in a glass homogenizer or by mortar and pestle in the presence of liquid nitrogen. The amount of material should be between about 1 mg and 1 gm, preferably between 10 mg and 250 mg, such as between 20 mg and 100 mg. The material to be sampled may be suspended in a suitable solvent, preferably phosphate-buffered saline at a pH between 7.0 and 7.8. The addition of RNase inhibitors is optional and preferred. The solvent may contain a detergent (*e.g*., Triton X-100, SDS, sarkosyl). Homogenization is performed for a number of excursions of the homogenizer, preferably between 10 and 25 strokes; such as between 15 and 20 strokes. The suspended sample is preferably centrifuged at between 100 and 1,000 x g for 5-10 minutes and the supernatant material sampled for analysis. In some samples, it may be preferable to treat the supernatant material with an additional reagent, such as phosphotungstic acid according to the procedure described by Safar et al., Nature Medicine 4:1157-1165, 1998, and as modified by Wadsworth, The Lancet 358:171-180, 2001.

The amount of sample to be tested is based on a determination of the protein content of the supernatant solution as measured by the procedure described by Bradford (Anal. Biochem. 72:248-254, 1976). A rapid and sensitive method for determining microgram quantities of protein utilizes the principle of protein-dye binding. Preferably, the amount of protein in the sample to be tested is between about 0.5 mg and 2 mg of protein.

In addition to the procedure described above for tissue material, test samples may be obtained from serum, pharmaceutical formulations that might contain products of animal origin, spinal fluid, saliva, urine, or other bodily fluids. Liquid samples may be tested directly or may be subjected to treatment with agents such as phosphotungstic acid, as described above.

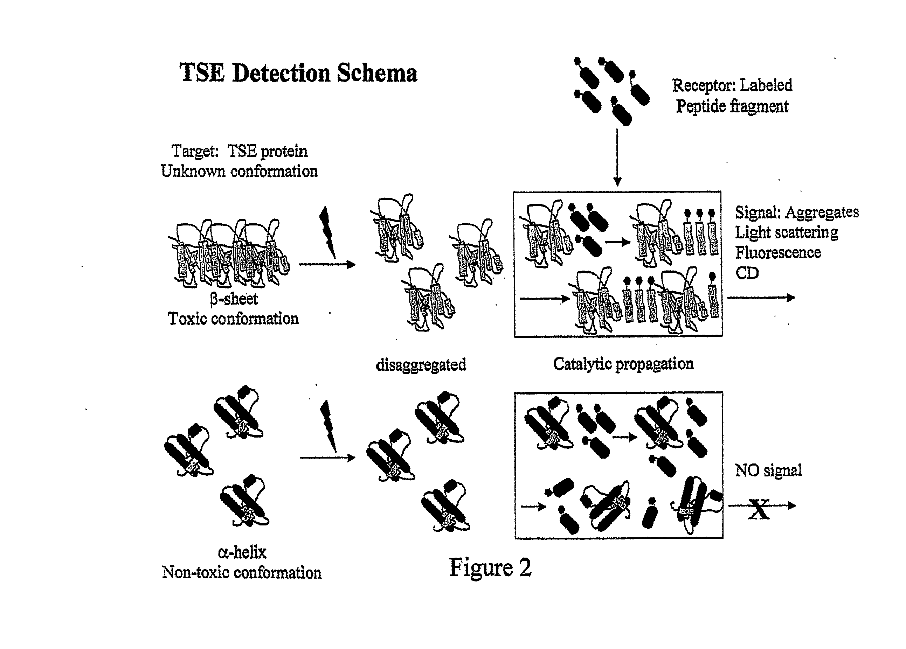

A sample containing TSE may be analyzed in accordance with the invention as follows. Referring to Figure 2, the top row of the schematic illustrates an unknown sample of TSE protein represented as containing beta-sheets. The beta-sheets are disaggregated by sonication. Labeled peptide probes are added and are allowed to bind to the sample. The beta-sheet conformation in the sample induces the peptide probes to conform to a beta-sheet conformation. Beta-sheet propagation among the peptide probes forms aggregates. The resulting transition to a predominantly beta-sheet form and amplified aggregate formation is detected by techniques such as light scattering and CD. In a particularly preferred embodiment, the peptide probe is fluorescently labeled and fluorescence detection is used.

The bottom row of Figure 2 shows an alternative example in which the unknown sample of TSE protein is represented in its normal alpha-helical form. For consistency, the sample is subjected to the same disaggregation process described above. Upon addition of the labeled peptide probes, neither a transition to beta-sheet form nor binding to the unknown samples occurs. As a result, there is no aggregate fluorescence signal in the case of a labeled peptide probe and there is no detection of aggregate formation by other analytical tools. Based on this schematic, unknown samples can be tested for the presence or absence of such abnormal protein conformations or sequences.

### Example 2

Polylysine was used as a model peptide. Experiments were performed using model systems to illustrate the conformational changes involved in the transition from a predominantly alpha-helix to a beta-sheet rich form. The model system chosen used a non-neurotoxic polyamino acid polylysine. The polyamino acid was chosen because of availability and safety. It normally evidences a random coil conformation at pH values between 5 and 9.

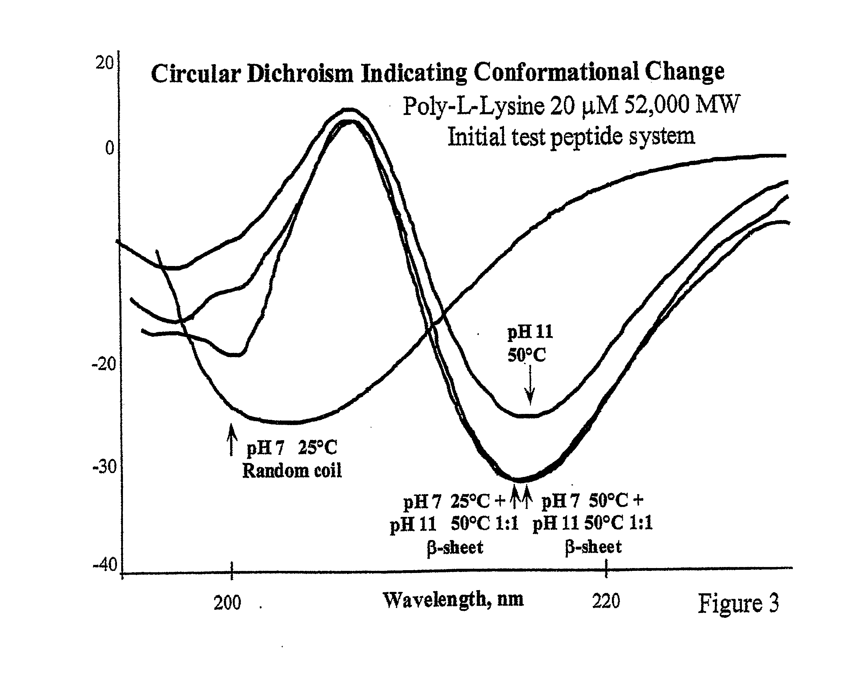

Figure 3 depicts a CD graph of an experiment in which poly-L-lysine (20 µM; 52,000 MW) was used as a peptide model.

As also illustrated in Figure 3: Sample 24, which was maintained at pH 7 and 25°C, exhibited a minimum at approximately 204 nm, indicating a random coil structure. Sample 26, which was maintained at pH 11 (near the isoelectric point) and 50°C, resulted in a minimum at approximately 216 nm, indicating a β-sheet structure (see Figure 11 for exemplary CD spectra of protein conformations). Sample 28, which was a 1:1 combination of samples maintained at pH 7 and 25°C, and at pH 11 and 50°C, resulted in a minimum at approximately 216 nm, indicating β-sheet structure. Sample 30, which was a 1:1 combination of samples maintained at pH 7 and 50°C, and at pH 11 and 50°C, resulted in a minimum at approximately 216 nm, indicating β-sheet structure.

### Example 3

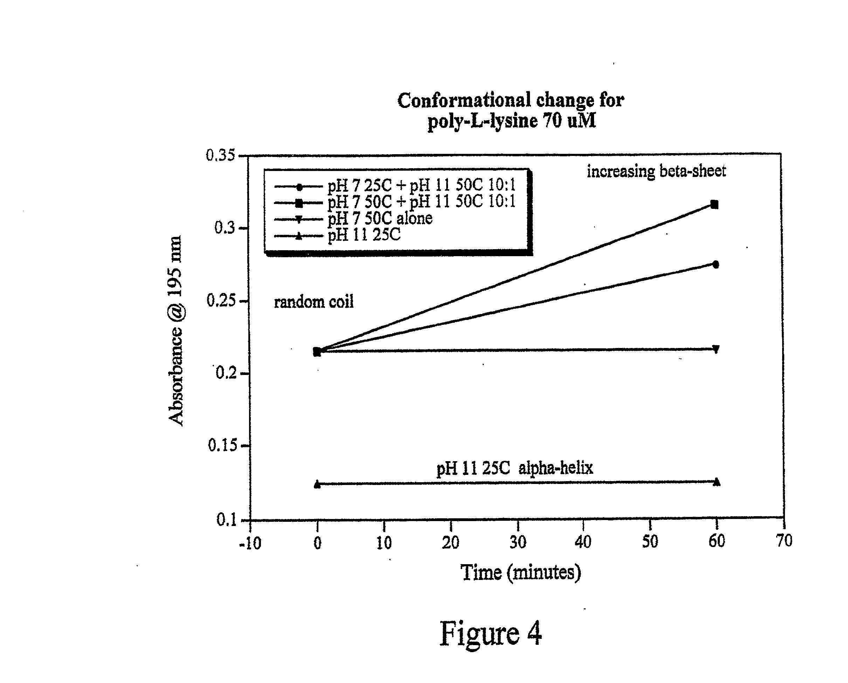

Figure 4 illustrates general CD results of experiments that were conducted:
(1) using poly-L-lysine; and (2) at varying temperatures and pH, to observe the effect of random coil to beta-sheet conformational changes under varying environmental conditions. The results indicate that both temperature and pH play an important role in the transition. The results also indicate that the addition of a relatively small amount of β-sheet peptide to a random coil sample can result in a shift towards a β-sheet rich conformation, and that such changes can be accelerated depending on the temperature and pH environment of the samples.

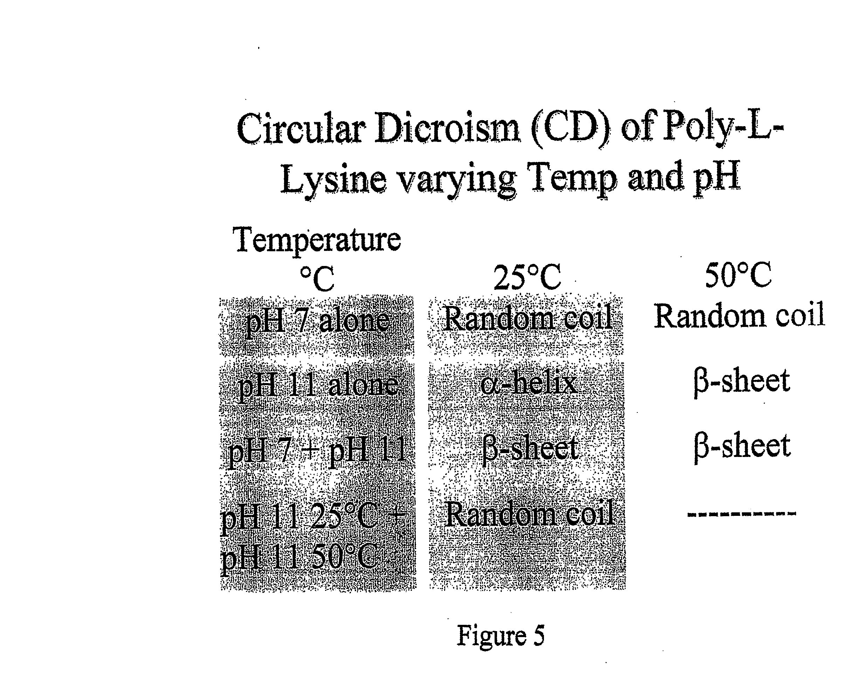

More specifically, Figure 4 illustrates an absorbance graph generated using a poly-L-lysine of 52,000 MW at 70 µM as a peptide probe in accordance with the experimental technique described in Examples 1-3. Figure 4 illustrates the following. Sample 32 (pH 11,25°C) evidenced a plateau at approximately 0.12, indicating a predominantly α-helical structure. Sample 34 (maintained at pH 7, 50°) evidenced a plateau at approximately 0.22, which indicated a predominantly random coil structure. Sample 36 (a 10:1 combination of samples maintained at pH 7, 50°C and at pH 11, 50°C) resulted in a steeper incline from approximately 0.22 to 0.33, indicating an accelerated transition from random coil to β-sheet structure. Sample 38 (a 10:1 combination of samples maintained at pH 7, 25° and at pH 11, 50°C) resulted in a gradual incline from approximately 0.22 to 0.26, indicating a transition from random coil to β-sheet structure. Figure 5 depicts these data in a tabular form.

The observations based on all of the experiments described above show that the addition of a relatively small amount of β-sheet peptide to random coil sample can result in a shift towards a beta-rich conformation and that such changes can be accelerated depending on the temperature and pH environment of the samples.

### Example 4

The experiment that led to the results illustrated in Figure 15 involved use of a 33-mer target peptide, which was either
VVAGAAAAGAVHKLNTKPKLKHVAGAAAAGAVV (murine) (SEQ ID NO:29), or
VVAGAAAAGAMHKMNTKPKMKHMAGAAAAGAVV (human) (SEQ ID NO:1); alone, and probed peptide association through the observation of excimer formation.

We compared the results we observed using CD (in which peptides were unlabeled) and spectrofluorometric studies (using pyrene-labeled peptides). No homogenate was used. The experiment that lead to the results illustrated in Figure 15 was a detailed study undertaken to understand what triggered the 33-mer target peptide (SEQ ID NO:1 or 29) to conformationally change from predominantly monomeric to dimeric (excimeric) and become aggregated. Conditions were found that encouraged 33-mer labeled-peptide association in the µM range.

Conditions that screened the electrostatic interactions of the 33-mer target peptide and thereby minimized its solubility (pI=10) triggered self-association of the peptide under extremely low concentrations of peptide (10 µM). This self association is evident in the formation of dimers or excimers and the concomitant far red shift in fluorescence by virtue of the pyrene fluorophor on the ends of the peptides. As an example, Curve 1 of Figure 15 represents the conditions of pH 6-8, KCI (100-500 µM) where the predominant peptide conformer is monomeric. Curve 2 of Figure 15 represents the conditions of pH 10-11, KCI (100-500 µM), where at very low concentrations of peptide, we observed strong excimer formation (aggregation of the monomers).

### Example 5

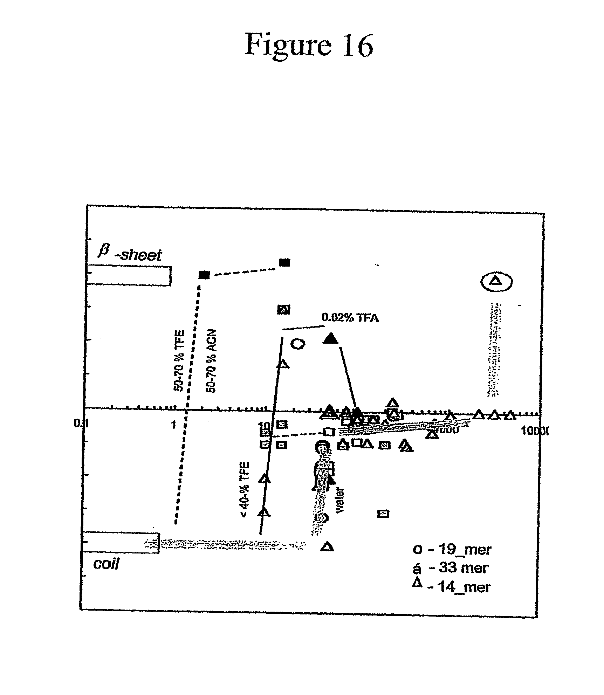

The experiment that led to the results illustrated in Figure 16 involved use of various individual peptides, and the 33-mer probe (comprising the 19-mer and 14-mer) target peptide (SEQ ID NO:1, 19, 2, or 3)
VVAGAAAAGAVHKLNTKPKLKHVAGAAAAGAVV (murine) (SEQ ID NO:29)
VVAGAAAAGAMHKMNTKPKMKHAGAAAAGAVV (human) (SEQ ID NO:1).
The assay conditions were changed to observe the effect on conformation as monitored by CD. The goal was to determine what thermodynamic conditions result in one step transition from monomeric random coil to aggregated β-sheet and avoid the associative "X" state that is probably micelle formation of the peptides.

In the experiment that lead to the results illustrated in Figure 16, a specific wavelength (204 nm) was used to monitor peptide association by CD to obtain detailed conformational information over a range of solvent conditions and across a range of peptide concentrations (peptide concentrations are presented in log scale and also refer to the standard diagram for CD - Figure 11).

The associative curve (θ₂₀₅) recovered for the target peptides showed two conformational transitions at the 50 µM and 3 mM range, respectively, moving from a coil through to the "X" state and to β**-**sheet.

Referring to Figure 16, for solvent conditions above 50% (far left dashed line), the 33-mer target peptide (SEQ ID NO:1 and 29)
VVAGAAAAGAVHKLNTKPKLKHVAGAAAAGAVV (murine) (SEQ ID NO:29)
VVAGAAAAGAMHKMNTKPKMKHMAGAAAAGAVV (human) (SEQ ID NO:1);
transitioned from the coil state to a β-sheet state at 3 µM, while the component 19-mer or 14-mer were able to transition, but at nearly 10-fold higher peptide concentrations (middle line). Under aqueous conditions (thick line), none of the peptides were able to self associate into a β-sheet structure.

The 33-mer palindromic peptide target peptide (SEQ ID NO: 1 and 29) exhibited unique properties at very low concentrations (*i.e.,* 1µM) under 50% solvent (acetonitrile or trifluoroethanol (TFE)) conditions in that it avoided the "dead-end" associative state (as exhibited by the plateauing effect under aqueous conditions).

Figure 16 shows that a variation in solvent and temperature does not significantly affect the associative behavior of target peptides and that all of the peptides follow the same curve, indicating that sequence specificity is not an important feature in this kind of molecular assembling.

### Example 6

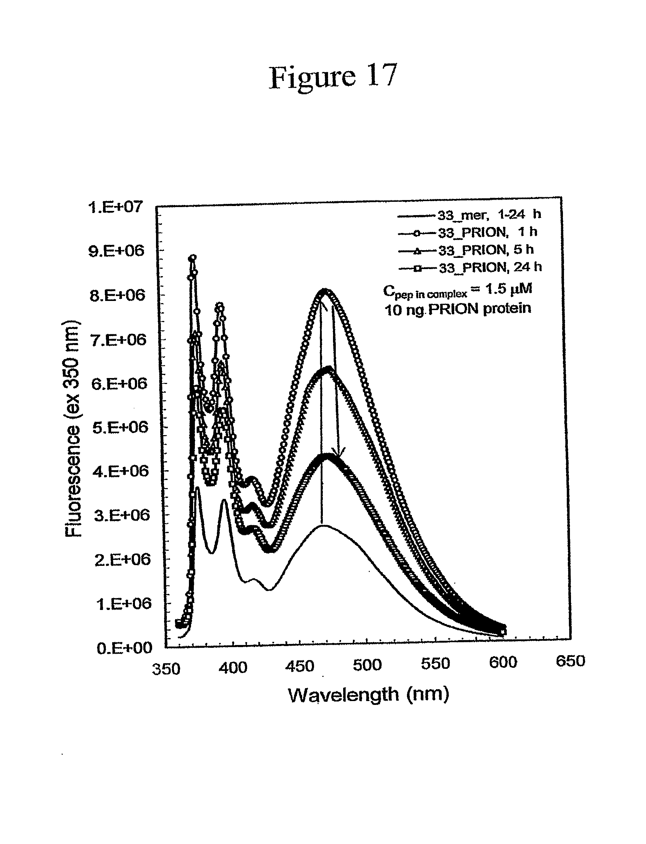

The experiment that led to the results illustrated in Figure 17 was conducted as follows:

One gram of scrapie infected (strain 293) hamster brain material was homogenized in liquid nitrogen in sterile phosphate buffered saline. Ten-fold serial dilutions were made into sterile PBS. The concentration of protease resistant prion protein (PrP^{Sc}) in the brain homogenates was determined by capillary electrophoresis antibody-capture. Brain homogenate equivalent to 10 ng of protease-resistant prion protein (PrP^{Sc}) was mixed with 1.5 µM of 33-mer target peptide in 50% TFE and incubated for 1 hour at room temperature prior to excitation at 350 nm in a dual chromometer spectrofluorometer and emission from 350 to 600 nm recorded, the excitation and emission scan was repeated at 5 hours and 24 hours. The 33-mer peptide alone was used as a control.

Addition of the infectious prion protein led to the significant increase in the fluorescence of the 33-mer target peptide, which was found to be in near β-sheet conformation by CD data under conditions of 50% Tris:50% TFE. This increase of fluorescence indicated the formation of 33-mer aggregates. The 33-mer aggregates were found to be unstable and dissociated irreversibly with time.

Following the emission of fluorescence for the complex versus the peptide over time illustrated that the complex dissociated with time, while the peptide fluorescence remained stable, as determined by monitoring at two different wavelengths, 377 nm (triangle) and 475 nm (square).

### Example 7

The experiment that led to the results illustrated in Figure 18 was conducted as follows:

One gram of scrapie infected and healthy hamster brain, sheep brain, and elk brain were homogenized in liquid nitrogen in sterile phosphate buffered saline. Ten-fold serial dilutions were made into sterile PBS. The concentration of protease resistant prion protein (PrP^{Sc}) in the brain homogenates was determined by capillary electrophoresis antibody capture. Brain homogenates, infected and healthy, were mixed with 0.52 µM of 33-mer target peptide in 50% TFE:50% Tris and incubated for 1 hour at room temperature prior to excitation at 350 nm in a dual chromometer spectrofluorometer, and emission at 350 to 600 nm recorded. The 33-mer peptide alone in 50% TFE:50% Tris was used as an additional control.

Fluorescence spectra of the target peptide (520 nM) in the presence of infected brain homogenate (graph, line 1), healthy brain homogenate (graph, line 2), and peptide alone (graph, line 3) in Tris:TFE (1:1) solvent are shown in Figure 18. The data are for 0.01% brain homogenate from hamster (panel A; 270 pg/ml), sheep (panel B; 60 pg/ml), and elk (panel C; 6 pg/ml).

### Example 8

Probes of the invention were immobilized on polystyrene plates to determine if particular probes could be immobilized and determined to be specific for different prion proteins. It was unexpectedly discovered that not only could the probes be immobilized, but that the immobilized probes retained their ability to bind to prion proteins, and their specificity for particular prion proteins.

It was known from previous experiments (discussed above) that probes of the invention (*e.g*., a 33-mer probe specific for human or murine prion proteins) will take on different conformations depending on the environment in which they are present, such as different pH, temperature, ionic strength. Thus, it was proposed that the different conformers would show different binding specificities. To investigate this, an attempt was made to immobilize probes having different conformations, and use these immobilized probes to test for the specificity of each conformation.

To obtain α-conformers the 33-mer target peptide was dissolved in neutral (pH about 7.0) phosphate buffered saline in the presence of 1% SDS. To obtain β-confonners, the peptide was dissolved in acidic (pH about 4.5 to about 5.0) citrate buffer (50 mM). The peptide solutions were incubated in a polystyrene plate overnight (∼16 hrs) until the specific conformer was immobilized onto the solid surface. Surprisingly, not only were the peptides immobilized to the plates in an active form (*i.e.,* able to bind to prion protein), but they retained their specific conformations, and thus maintained their specificity even when immobilized. Even more surprising, the immobilized 33-mers maintained their specific conformations even when the buffering conditions were changed at a later time. That is, a probe that was designed and immobilized under conditions that resulted in predominantly β-sheet probe conformation maintained that conformation after immobilization even if the conditions to which it was exposed were altered to favor formation of the α-helical conformation.

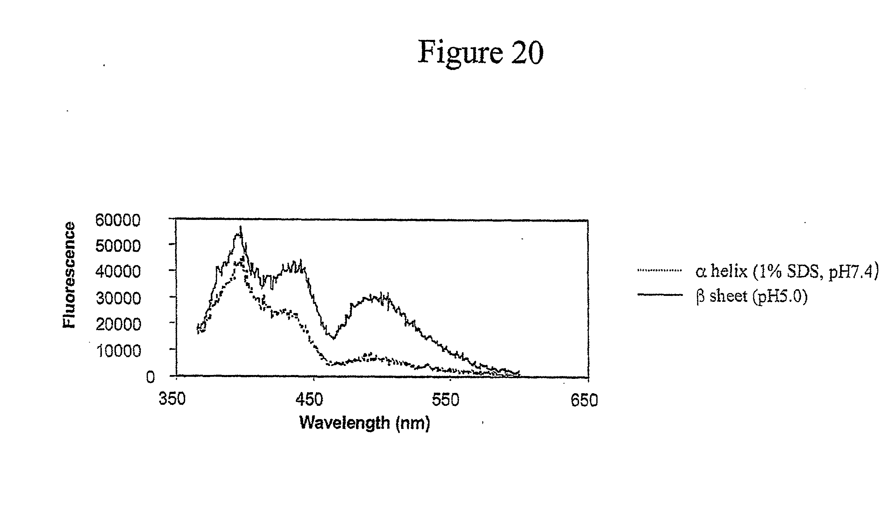

Figure 20 depicts the combined results of independent immobilization of an α-helix 33-mer probe and a β-sheet 33-mer probe. The probes were labeled with pyrene and independently immobilized onto two different plates under the conditions described above. After removal of the specific buffers, the immobilized peptides were re-incubated with phosphate-buffered saline (pH 7.0), and a fluorescence spectroscopy was performed on the plate immobilized with either α- or β-conformers. Under the α-conformer forming condition (PBS, pH7.0, 1% SDS), there is no peak around 500 nm (dotted line), while under the β-conformer forming condition, the immobilized 33-mer peptide showed a peak around 500 nm (solid line). The appearance of this peak around 500 nm is an indicator for the presence of 33-mer excimers that are mainly β-conformers in solution. Thus, even though the immobilized β-conformer probe was subjected to conditions where α-conformers are formed, it retained its β-conformation.

### Example 9

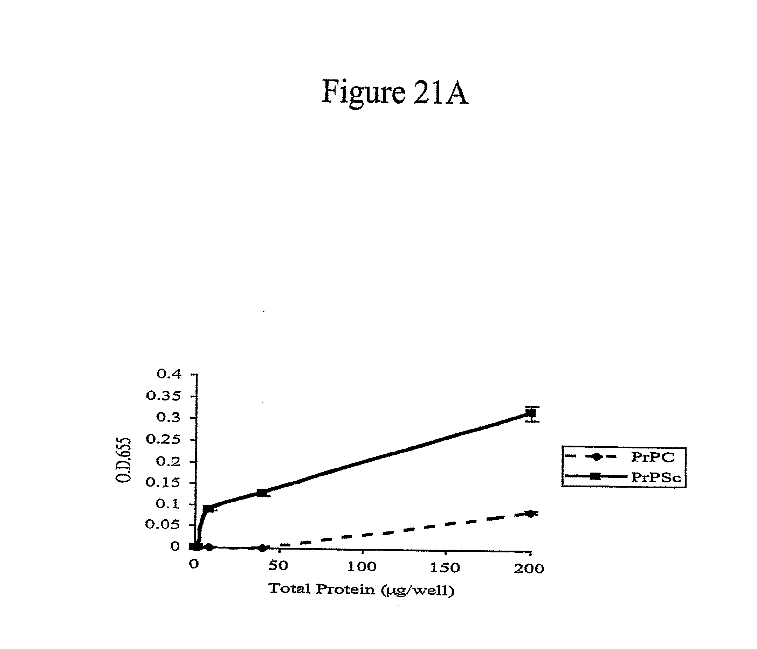

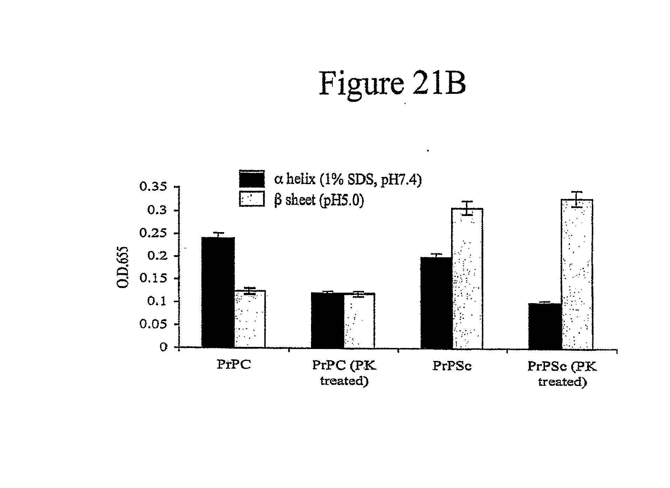

The different conformers of a peptide probe can bind PrP^{C} and PrP^{Sc} with different specificities. For example, the 33-mer probes in the β-conformation specifically bind PrP^{Sc}, whereas the 33-mer probes in the α-conformation preferentially bind PrP^{C}. This fact was confirmed using immobilized probes in both the α-conformation and β-conformation, and data supporting it is shown in Figure 21.

It has been found that certain conformers (*i.e*., β-conformers) can specifically bind PrP^{Sc}, and the bound PrP^{Sc} can be further detected by other techniques, such as by detection with antibodies specific for the particular conformer. To demonstrate this fact, brain lysates containing different amounts of total proteins from both normal (dashed line in Figure 21A) and scrapie infected brain (solid line in Figure 21A) were incubated with either α- or β-conformers of the 33-mer peptide (SEQ ID NO:1 or 29) immobilized onto a polystyrene plate. After incubation of 1-2 hrs at room temperature, non-specifically bound substances were washed away with PBS containing 0.05 % Tween-20, and the bound PrP was detected by anti-PrP monoclonal antibodies. As shown in Figure 21A, when a β-conformer probe is immobilized to the plate and exposed to a sample containing both PrP^{Sc} and PrP^{C}, the probe specifically binds to PrP^{Sc} at low protein concentrations (less than about 50 µg), and preferentially binds to PrP^{Sc} at higher protein concentrations (about 50 µg or higher).

The experiment depicted in Figure 21B shows that the immobilized probes distinguish between PrP^{Sc} and PrP^{C}, the β-conformer preferentially binding to PrP^{Sc} and the α-conformer preferentially binding to PrP^{C}. Specifically, the two probe conformers were independently bound to different plates, as detailed above. The bound probes were exposed to brain lysates containing both PrP^{Sc} and PrP^{C}, and the amount of prion protein bound to each probe was determined (indicated by bars labeled "PrPC" and "PrPSc"). A comparison of these two experiments shows that the α-helical conformer probe binds PrP^{C} and, to a lesser degree PrP^{Sc}, whereas the β-sheet conformer probe binds only PrP^{Sc} to any significant extent. When the brain lysate was treated with proteinase K, the proteinase K resistant PrP was detected only to bind the β-conformer probe (compare bars labeled "PrPC (PK treated)" and PrPSc (PK treated)"). This data indicated that the β-conformer can selectively bind to PrP^{Sc}.

These results have applicability to a wide range of technical and industrial endeavors. For example, the results indicate that the β-conformer can be chemically coupled onto solid supports, such as membranes and gel matrices, then used in an aqueous solution of neutral pH, low salt, or with or without additives, to specifically bind and remove PrP^{C}, PrP^{Sc}, or both. The data indicate that clearing infective prion proteins from blood or blood products (*e.g*., plasma) can be accomplished by binding a β-conformer to a membrane, passing the blood or blood product through the probe-immobilized membrane, and separating the membrane from the blood or blood product. Indeed, it is evident that one can use an immobilized β-conformer probe alone to remove infective prion particles, or one can use a mixture of β-conformer and α-conformer probes to remove all prion particles from a sample, such as a blood or blood product sample.

### Example 10

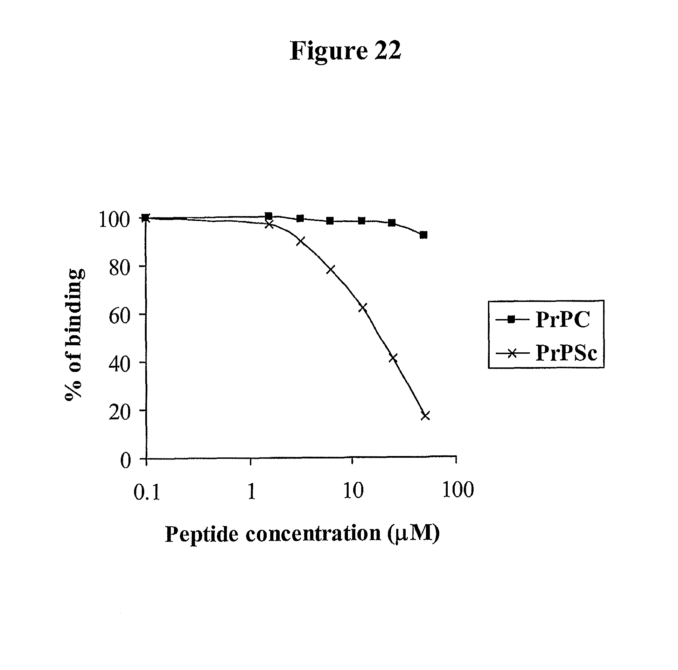

This Example shows that peptide bound to a solid support in a predominantly β-sheet form specifically binds mis-folded prion protein (PrP^{Sc}) as compared to normal prion protein (PrP^{C}). More specifically, a peptide comprising the sequence of SEQ ID NO: 1 was treated with an acidic citrate buffer (50 mM citrate, pH about 4.5 to about 5.0) and bound to a microtiter plate well (4 mg/ml peptide; 0.1 ml/well) overnight. Then the coated plate was incubated with brain homogenates (10%) from either normal or scrapie infected hamsters. In addition, various amounts of free peptide of identical sequence to the probe, but either in predominantly α-helical form or predominantly β-sheet form, were incubated with the brain homogenate, to determine the I₅₀ or K_{I} of the binding between PrP^{Sc} or PrP^{C} and the bound peptide. Bound prion proteins were detected by anti-PrP monoclonal antibodies, and the results are depicted in Figure 22. The binding (Y axis) was expressed as the percentage of maximal binding (without the presence of free peptide). It is thus a relative scale, the total amount of each conformation of prion protein being bound to the solid-support bound probe at a different level.

As can be seen from Figure 22, bound probe that is locked in a predominantly β-sheet conformation specifically binds to PrP^{Sc}, as compared to PrP^{C}. That is, binding of PrP^{Sc} can be competed away from the bound β-sheet probes by probes comprising prion sequences in predominantly β-sheet conformation, but not by probes comprising prioin sequences in predominantly α-helix conformation.

It will be apparent to those skilled in the art that various modifications and variations can be made in the practice of the present invention without departing from the scope or spirit of the invention. Other embodiments of the invention will be apparent to those skilled in the art from consideration of the specification and practice of the invention. It is intended that the specification and examples be considered as exemplary.

In view of the foregoing, it will be appreciated that the invention described herein *inter alia* relates to the following items:
1. A method of immobilizing a prion protein specific probe to a solid support, said method comprising
   providing a first prion protein specific peptide probe in a pre-selected conformation;
   providing a solid support; and
   exposing the solid support to the first probe under conditions that maintain the first probe in its pre-selected conformation and under conditions that permit immobilization of the first probe to the solid support, for a sufficient amount of time for the first probe to be immobilized on the solid support.
2. The method of item 1, further comprising removing any probe that is not immobilized on the solid support.
3. The method of item 1, further comprising, prior to exposing the solid support to the first probe:
   exposing the first probe to a composition comprising a prion protein for a sufficient amount of time and under suitable conditions for the first probe and prion protein to contact each other.
4. The method of item 3, wherein the prion protein is in a mis-folded conformation.
5. The method of item 4, wherein a complex between the prion protein and first probe is formed, and wherein the first probe is immobilized on the solid support either directly through binding of the first probe to the solid support or indirectly through
   binding of the first probe or the prion protein to a second probe, wherein the second probe is immobilized on the solid support.
6. The method of item 1, wherein the first probe comprises the sequence of SEQ ID NO:1 or SEQ ID NO:29.
7. The method of item 1, wherein the conformation of the first probe is the β-conformation.
8. The method of item 1, wherein the first probe is immobilized directly to the solid support.
9. The method of item 1, further comprising immobilizing a third prion protein specific peptide probe to the solid support by
   providing a third prion protein specific peptide probe in a pre-selected conformation;
   providing the solid support comprising the first probe;
   exposing the solid support to the third probe under conditions that maintain the third probe in its pre-selected conformation and under conditions that permit immobilization of the third probe to the solid support, for a sufficient amount of time for the third probe to be immobilized on the solid support; and
   optionally removing any probe that is not immobilized.
10. The method of item 1, wherein the solid support comprises plastic, latex, or metal.
11. The method of item 1, wherein the solid support is in the form of a plate, a bead, or a membrane.
12. The method of item 1, wherein the first probe is labeled.
13. A method of binding a prion protein to a probe immobilized on a solid support, said method comprising
   providing an immobilized probe having a pre-determined conformation and specificity for a prion protein;
   providing a sample containing or suspected of containing a prion protein; and exposing the sample to the immobilized probe under conditions and for an amount of time sufficient for the immobilized probe to bind to a prion protein in the sample.
14. The method of item 13, further comprising detecting the presence of a prion protein bound to the immobilized probe.
15. The method of item 13, wherein the method is used to screen animal or human food, or animal or human medical products, for the presence of a prion protein.
16. The method of item 13, wherein the method is used to screen animal or human food, or animal or human medical products, for the presence of PrP^{Sc}.
17. The method of item 13, wherein the sample comprises animal or human blood or blood products.
18. The method of item 13, wherein the probe comprises the sequence of SEQ ID NO:1 or SEQ ID NO:29.
19. The method of item 13, wherein the pre-determined conformation is the β-conformation of the probe.
20. The method of item 13, further comprising separating the immobilized probe and immobilized probe-prion protein complexes from the sample.
21. The method of item 20, wherein the method is a method of removing a prion protein from a sample.
22. The method of item 20, wherein the method reduces or eliminates prion proteins from an animal or human food or animal or human medical product.
23. The method of item 20, wherein the prion protein is PrP^{Sc}.
24. The method of item 20, wherein the sample comprises animal or human blood or blood products.
25. The method of item 20, wherein the probe comprises the sequence of SEQ ID NO:1 or SEQ ID NO:29.
26. The method of item 20, wherein the pre-determined conformation is the β-conformation of the probe.
27. A method of binding a prion protein to a probe immobilized on a solid support, said method comprising
   providing a probe that is immobilized on a solid support and that specifically binds to a prion protein when the probe is in its immobilized state;
   providing a sample containing a prion protein; and
   exposing the sample to the immobilized probe under conditions and for an amount of time sufficient for the immobilized probe to bind to a prion protein in the sample.
28. The method of item 27, further comprising, prior to exposing the sample to the immobilized probe:
   providing a second probe that specifically binds to a prion protein; and
   exposing the second probe to the sample for a sufficient amount of time and under suitable conditions for the second probe to bind to a prion protein present in the sample.
29. The method of item 28, wherein the second probe is labeled with a detectable label.
30. A kit comprising, in packaged combination, at least one container containing at least one probe immobilized on a solid support, wherein the immobilized probe has a pre-determined conformation and binds to a known prion protein or set of prion proteins, and wherein the pre-determined conformation of the immobilized probe does not change when the immobilized probe is exposed to conditions that cause a change in the probe when not immobilized.
31. The kit of item 30, wherein the immobilized probe comprises the sequence of SEQ ID NO:1 or SEQ ID NO:29.
32. The kit of item 30, wherein the pre-determined conformation is the β-conformation of the probe.
33. The kit of item 30, wherein the kit further comprises a second probe, which is not immobilized to a solid support, and which binds to a prion protein.
34. The kit of item 33, wherein the second probe comprises the sequence of SEQ ID NO: 1 or SEQ ID NO:29.
35. A method of immobilizing a conformational peptide probe for a target protein associated with an amyloidogenic disease to a solid support, comprising:
   a) providing a first peptide probe in a random coil/alpha helix conformation or a β-sheet conformation and consisting of from 10 to 50 amino acid residues comprising
      (i) an amino acid sequence corresponding to a β-sheet forming region of SEQ ID NO:4, wherein said amino acid sequence has an amino acid sequence identity of at least 40%, 50%, 70%, 90%, 99% or 100% to said β-sheet forming region of SEQ ID NO:4; and
   b) exposing the first peptide probe to a solid support under conditions that
      (i) maintain the first peptide probe in said conformation and
      (ii) permit immobilization of the first peptide probe to the solid support, for a time period such that the first conformational peptide probe is immobilized to the solid support in that conformation.
36. The method of item 35, wherein the peptide probe comprises the amino acid sequence of any one of SEQ ID NO:5, SEQ ID NO:6 or SEQ ID NO:7.
37. The method of item 35, wherein the conformation of the conformational peptide probe is a random coil/alpha-helix conformation.
38. The method of item 35, wherein the conformation of the conformational peptide probe is a β-sheet conformation.
39. The method of any one of the preceding items, further comprising removing any conformational peptide probe that is not immobilized to the solid support.
40. The method of any one of the preceding items, further comprising immobilizing a second conformational peptide probe to the solid support.
41. The method of item 40, wherein the second conformational peptide probe is immobilized to the solid support by a method comprising:
   a) providing a second conformational peptide probe;
   b) exposing the solid support to the second conformational peptide probe under conditions that
      (i) maintain the second conformational peptide probe in the conformation and
      (ii) permit immobilization of the second conformational peptide probe to the solid support, for a time period such that the second conformational peptide probe is immobilized to the solid support in the conformation; and c) optionally, removing any second conformational peptide probe that is not immobilized to the solid support.
42. The method of any one of the preceding items, wherein the conformational peptide probe comprises palindromic amino acid sequences comprising:
   (a) a first amino acid sequence corresponding to a β-sheet forming region of the target protein, and
   (b) a second peptide sequence that is the reverse of the first amino acid sequence.
43. The method of item 42, further comprising a peptide linker between the first and the second amino acid sequences.
44. The method of item 43, wherein the peptide linker comprises a proline residue.
45. A method of binding a target protein associated with an amyloidogenic disease to a conformational peptide probe immobilized on a solid support, comprising:
   contacting a sample containing or suspected of containing a target protein with said conformational peptide probe for a period of time such that the immobilized conformational peptide probe binds to any target protein in the sample;
   wherein the conformational peptide probe is immobilized on the solid support according to any one of the preceding items.
46. The method of any one of the preceding items, further comprising detecting the presence of any target protein bound to the immobilized conformational peptide probe.
47. The method of items 45 or 46, further comprising separating the immobilized conformational peptide probe, and any target protein bound thereto, from the sample.
48. The method of any one of items 45 to 47, wherein the sample is a biological sample.
49. The method of any one of items 45 to 48, wherein the sample is selected from the group consisting of tissue, meat, a biopsy sample, blood, a blood fraction, plasma, serum, pharmaceutical formulations that might contain products of animal origin, spinal fluid, saliva, urine, bodily fluids, food products, and medical products.
50. The method of any one of the preceding items, wherein the solid support comprises a material selected from the group consisting of plastics, glass, polysaccharides, metal and polymers.
51. The method of any one of the preceding items, wherein the solid support is in the form of a plate, a bead, or a membrane.
52. The method of any one of the preceding items, wherein the target protein is associated with Alzheimer's Disease.
53. The method of any one of the preceding items, wherein the conformational peptide probe comprises an amino acid sequence containing at least 75% hydrophobic amino acids.

## Claims

1. A method of immobilizing a conformational peptide probe for a target protein associated with an amyloidogenic disease to a solid support, comprising:
a) providing a first peptide probe in a random coil/alpha helix conformation or a β-sheet conformation and consisting of from 10 to 50 amino acid residues comprising
(i) an amino acid sequence corresponding to a β-sheet forming region of SEQ ID NO:4, wherein said amino acid sequence has an amino acid sequence identity of at least 40%, 50%, 70%, 90%, 99% or 100% to said β-sheet forming region of SEQ ID NO:4; and
b) exposing the first peptide probe to a solid support under conditions that
(i) maintain the first peptide probe in said conformation and
(ii) permit immobilization of the first peptide probe to the solid support, for a time period such that the first conformational peptide probe is immobilized to the solid support in that conformation.

2. The method of claim 1, wherein the peptide probe comprises the amino acid sequence of any one of SEQ ID NO:5, SEQ ID NO:6 or SEQ ID NO:7.

3. The method of claim 1, wherein the conformation of the conformational peptide probe is a random coil/alpha-helix conformation.

4. The method of claim 1, wherein the conformation of the conformational peptide probe is a β-sheet conformation.

5. The method of any one of the preceding claims, further comprising removing any conformational peptide probe that is not immobilized to the solid support.

6. A method of binding a target protein associated with an amyloidogenic disease to a conformational peptide probe immobilized on a solid support, comprising:
contacting a sample containing or suspected of containing a target protein with said conformational peptide probe for a period of time such that the immobilized conformational peptide probe binds to any target protein in the sample;
wherein the conformational peptide probe is immobilized on the solid support according to any one of the preceding claims.

7. The method of any one of the preceding claims, further comprising detecting the presence of any target protein bound to the immobilized conformational peptide probe.

8. The method of claims 6 or 7, further comprising separating the immobilized conformational peptide probe, and any target protein bound thereto, from the sample.

9. The method of any one of claims 6 to 8, wherein the sample is a biological sample.

10. The method of any one of claims 6 to 9, wherein the sample is selected from the group consisting of tissue, meat, a biopsy sample, blood, a blood fraction, plasma, serum, pharmaceutical formulations that might contain products of animal origin, spinal fluid, saliva, urine, bodily fluids, food products, and medical products.

11. The method of any one of the preceding claims, wherein the solid support comprises a material selected from the group consisting of plastics, glass, polysaccharides, metal and polymers.

12. The method of any one of the preceding claims, wherein the solid support is in the form of a plate, a bead, or a membrane.

13. The method of any one of the preceding claims, wherein the target protein is associated with Alzheimer's Disease.

14. The method of any one of the preceding claims, wherein the conformational peptide probe comprises an amino acid sequence containing at least 75% hydrophobic amino acids.

15. A method of immobilizing a peptide probe to a solid support, comprising:
providing a first peptide probe in a pre-selected conformation; and
exposing the first peptide probe to a solid support under conditions that
(i) maintain the first peptide probe in its pre-selected conformation and
(ii) permit immobilization of the first peptide probe to the solid support, for a time period sufficient for the first peptide probe to be immobilized to the solid support.

16. A method of binding a target protein to a peptide probe immobilized on a solid support, comprising:
contacting a sample containing or suspected of containing a target protein with a peptide probe immobilized to a solid support in a pre-selected conformation for a period of time sufficient for the peptide probe to bind to any target protein in the sample.
